# NEW EUROPEAN PATENT SPECIFICATION

(11) **EP 0 430 287 B2**
(45) Date of publication and mention of the opposition decision: **12.07.2000**
(45) Mention of the grant of the patent: 12.10.1994
(21) Application number: 90122985.6
(22) Date of filing: 30.11.1990
(51) Int. Cl.: A61K 9/54, A61K 9/52, A61K 9/20

(54) **Sustained-release drug dosage units**
Einzeldosen mit verzögerter Arzneistofffreigabe
Doses unitaires pour la libération prolongée d'un médicament

(30) Priority: 01.12.1989 US 444458; 02.11.1990 US 605152
(43) Date of publication of application: 05.06.1991
(73) Proprietor: ABBOTT LABORATORIES, Abbott Park, Illinois 60064-3500 (US)
(72) Inventor: Brinker, Dale R., Antioch, Illinois 60002 (US); Eveline, Enrique D., Waukegan, Illinois 60085 (US)
(74) Representative: Modiano, Guido, Dr.-Ing.

(56) References cited:
- EP-A- 0 014 514
- EP-A- 0 080 341
- EP-A- 0 235 986
- EP-A- 0 347 748
- US-A- 4 131 473
- Drug Development and Industrial Pharmacy, 15(10), 1989, pp. 1495-1521
- Eudragit L and S - Prospectus (infor L/S-1/e) - Rohm Pharma - 1984
- Pharmaceutical Technology, April 1984, pp. 64-71
- Eudragit RL/RS (info RL/RS - 12/e) - Rohm Pharma, 1983
- "Coating Conditions - Aquacoat Aqueous polymeric dispersion" - FMC Corporation, 1982
- "Update 4 - Aquacoat Aqueous polymeric dispersion" FMC Corporation, 1985
- Int. J. Pharm. Tech. & Prod. Mfr. 5(8), 1-9, 1984

## Description

This is a continuation-in-part of U.S. patent application Serial No. 444,458, filed December 1, 1989, which is a continuation-in-part of U.S. patent application Serial No. 353,809, filed May 22, 1989 (abandoned), which is a continuation-in-part of U.S. patent application Serial No. 211,495, filed June 24, 1988 (abandoned).

### Technical Field

The present invention relates to sustained-release drug dosage units. Compositions and methods for preparing sustained-release dosage units are described.

### Background of the Invention

The use of sustained-release drug dosage units for the oral administration of drugs to a patient has several therapeutic advantages. A therapeutically effective systemic level of a drug can be maintained over an extended time period without the necessity of multiple daily drug administration. Some drugs are toxic or otherwise deleterious in high concentrations, and thus require multiple administration of low-level amounts of these drugs to patients. Such administration results in a bolus rise in drug concentration initially, above the most effective therapeutic level, and a concomitant decrease over time below this level, producing a high-low fluctuation in blood levels of the drug. Sustained-release dosage forms overcome this problem by releasing the drug in small amounts throughout a predetermined time period and thus maintaining the drug level in the blood within a narrow therapeutically effective range as the rate of drug release and systemic drug removal are maintained in balance.

Numerous methods and compositions for preparing controlled-release drug compositions are known, and include those described in U.S. Patent No. 4,572,833 to Pedersen, et al.; U.S. Patent Nos. 4,713,248 and 4,716,041 to Kjornaes, et al; U.S. Patent No. 4,772,475 to Fukui, et al.; U.S. Patent No. 4,756,911 to Drost, et al.; U.S. Patent No. 4,786,506 to Fontanelli; U.S. Patent No. 4,351,825 to Southman, et al; U.S. Patent No.4,252,786 to Weiss, et al.; U.S. Patent No. 4,199,560 to Gyarmati, et al; and Colombo, et al., European Patent Application No. EP0092060.

EP-A-0 347 748 which is comprised in the state of the art under the terms of Article 54(3) and (4) EPC, discloses pharmaceutical granules and tablets made therefrom. Disclosed therein are tablets comprising granules consisting of divalproex sodium and silica gel coated with a composition consisting of ethylcellulose, triethyl citrate and magnesium stearate.

### Summary of the Invention

The present invention is directed to a sustained-release drug coating composition, sustained-release coated drug granules, sustained-release drug dosage units and methods for the preparation and administration of a sustained-release drug dosage unit.

More particularly, the present invention is directed to drug granules coated with a composition which slows drug release. These coated drug granules are mixed with polymeric materials to form a dosage unit that further slows release of the drug. The resulting composition provides a continuous slow release of a therapeutically effective level of drug in the patient.

The sustained-release drug coating composition of the present invention comprises an ethylcellulose or a methacrylic methylester, a plasticizer and a detackifying agent.

Preferably, the sustained-release drug coating composition of the present invention comprises from 2 to 20 weight percent (g/ml x 100) of an ethylcellulose and/or a methacrylic methylester, from 0.1 to 5.0 weight percent (g/ml x 100) of a plasticizer and from 0.5 to 20 weight percent (g/ml x 100) of a detackifying agent.

A drug dosage unit of the present invention contains sustained-release coated drug granules together with at least one viscosity agent such as methylcellulose, hydroxypropylmethylcellulose, povidone, hydroxypropylcellulose, and the like. The dosage unit is preferably a tablet formed by compression of this mixture.

A method of treatment of the present invention comprises orally administering a drug dosage unit of the present invention to a patient in need of such treatment.

The present invention is also directed to a method for the preparation of a sustained-release drug dosage unit. In this method, a drug is first admixed with a detackifying agent such as silica gel and wetted with a solvent such as ethanol with further mixing to form drug granules. The drug granules are then dried and sized, such as by sifting through screens, to obtain granules of a desired size; larger granules are then milled and sized to produce more drug granules of the desired size. The appropriately sized granules are then admixed with a sustained-release drug coating composition of the present invention to produce coated drug granules. The coated drug granules are then mixed with at least one viscosity agent of the present invention and formed into sustained-release drug dosage units such as tablets.

The present invention is also directed to a method for the preparation of a sustained-release drug dosage unit wherein a solution of the drug is coated on sugar spheres. The resulting drug granules are then admixed with a sustained-release coating composition of the present invention to produce coated drug granules. The coated drug granules are then mixed with at least one viscosity agent of the present invention and formed into sustained-release drug dosage units such as tablets.

### Description of Preferred Embodiments

The present invention is directed to the sustained release of a drug over a predetermined time period following adminstration to a patient. A drug is formulated into granules and then coated with a coating composition that contains an ethylcellulose and/or a methacrylic methylester together with plasticizer and a detackifying agent dissolved or dispersed in a solvent such as ethanol and/or acetone.

As used herein, the term "coating composition" refers to a mixture of designated compounds that when applied to granules of a drug produces an insoluble coating through which the drug is slowly released.

As used herein, the terms "an ethylcellulose" and "a methacrylic methylester" refer to both substituted and unsubstituted forms of ethylcellulose and methacrylic methylester, respectively, and include ethylcellulose, hydroxypropylethylcellulose, methacrylic methylester, polymethylmethacrylate, and the like.

As used herein, the term "plasticizer" refers to a component of the coating composition that has a low vapor pressure and whose presence in the composition modifies the flexibility and diffusion properties of the coating composition.

As used herein, the term "detackifying agent" refers to a compound whose presence in the coating composition reduces the stickiness or adhesion of the coated drug granules.

Plasticizers useful in the coating composition can include castor oil, propylene glycol, polyethylene glycol, acetyl triethyl citrate, acetyl tributyl citrate, triethyl citrate, tributyl citrate, and the like. Plasticizers are present in the coating composition of the present invention at a concentration of about 0.1 to about 5.0 weight percent (w/v) of the total weight of the composition.

Detackifying agents useful in the present invention include magnesium stearate, talc, titanium dioxide, silica gel, and the like. The detackifying agent is present in the coating composition of the present invention at a concentration of about 0.5 to about 20 weight percent (w/v) of the total weight of the composition.

Drugs useful in the present invention are orally administerable drugs and preferably include divalproex sodium and valproic acid, its salts or derivatives thereof. Other drugs useful in the present invention include terazosin (1-(4-amino-6,7-dimethoxy-2-quinazolinyl)-4-((tetrahydro-2-furanyl)carbonyl)-piperazine), its salts, hydrates or derivatives thereof.

Derivatives of valproic acid include amides and esters thereof. Esters of valproic acid include the 2-propylpentanol-di-n-propylacetate and glycerol tri-(dipropylacetate).

In a preferred embodiment, granules of divalproex sodium or terazosin hydrochloride dihydrate are coated with a composition containing ethylcellulose, castor oil and magnesium stearate in a solvent mixture such as ethanol and acetone.

In a particularly preferred embodiment, a sustained-release drug particle composition of the present invention comprises from 62 to 86 weight percent divalproex sodium, from 2.6 to 3.7 weight percent silica gel, from 2.6 to 11.6 weight percent ethylcellulose, from 0.4 to 1.9 weight percent castor oil, from 2.6 to 11.6 weight percent magnesium stearate, from 0 to 0.09 weight percent edible dye, from 0.4 to 1.0 weight percent povidone and from 4.5 to 9.7 weight percent microcrystalline cellulose.

In another particularly preferred embodiment, a sustained-release drug particle composition of the present invention comprises about 12.9 weight percent terazosin monohydrochloride dihydrate, about 5.5 weight percent povidone, about 53.6 weight percent sugar spheres, about 8.0 weight percent ethylcellulose, about 1.3 weight percent castor oil, about 8.0 weight percent magnesium stearate and about 10.7 weight percent microcrystalline cellulose.

The sustained-release coated drug granules of the present invention are formed into drug dosage units, such as tablets, by admixing them together with at least one viscosity agent, and preferably with two viscosity agents, prior to forming the composition into tablets. Illustrative viscosity agents include methylcellulose, hydroxypropylmethylcellulose, povidone and hydroxypropylcellulose.

As used herein, the term "drug dosage unit" refers to a dosage form that is capable of being orally administered to produce a sustained release of a drug therefrom. Illustrative drug dosage units include tablets, capsules and lozenges.

As used herein, the term "viscosity agent" refers to an ingredient of a drug dosage unit which forms an insoluble or non-disintegrating polymeric matrix in which the coated drug granules of the present invention are enclosed, and from which the drug is slowly released.

A preferred drug dosage unit of the present invention contains about 2.0 to about 80 weight percent of sustained-release coated drug granules, about 0 to about 35 weight percent of calcium phosphate, about 2 to about 30 weight percent of a first viscosity agent and about 0 to about 30 weight percent of a second viscosity agent.

In a particulary preferred embodiment, the drug granules contain divalproex sodium, and the dosage unit contains from about 63 to about 65 weight percent of sustained-release coated granules of divalproex sodium, from about 3.5 to about 4.5 weight percent of methylcellulose, from about 14.2 to about 14.6 weight percent of hydroxypropylmethylcellulose, from about 14.2 to abut 14.6 weight percent of dibasic calcium phosphate, from about 0.7 to about 0.8 weight percent of stearic acid and from about 1.5 to about 2.2 weight percent of talc.

In another particularly preferred embodiment, the drug granules contain terazosin monohydrochloride dihydrate, and the dosage unit contains from about 2.6 to about 26.4 weight percent of sustained-release coated granules of terazosin hydrochloride dihydrate, about 2.5 weight percent of methylcellulose, about 10.5 weight percent of hydroxypropylmethylcellulose, about 34.0 weight percent of dibasic calcium phosphate, about 1.1 weight percent of stearic acid and about 1.9 weight percent of talc.

The present invention is also directed to a method of treatment in which a sustained-release drug dosage unit is administered to a patient in need of such treatment for the sustained release of a drug such as divalproex sodium or terazosin. In this method, a drug dosage unit of the present invention is orally administered to a patient, to release the drug systemically over a period of about 10 to about 12 hours.

A drug dosage unit of the present invention is prepared by admixing a drug, such as divalproex sodium or valproic acid, it salts or derivatives thereof, or terazosin, its salts, hydrates or derivatives thereof, with a detackifying agent such as silica gel in a solvent, such as ethanol, to produce drug granules. The drug granules are then dried, such as by warm air, and sized, such as by sifting through screens, to obtain granules of a desired size. Large granules are further milled and sized to obtain granules of the desired size. These granules are then mixed with a coating composition of the present invention to produce coated granules, which are then mixed with a composition containing at least one viscosity agent, and then formed into an appropriate drug dosage unit, such as tablets.

As used herein, the term "sizing screen" refers to a screen having openings of a definite specified size over which a mixture of solid particles, such as drug granules, is placed to fractionate the particles by size. Particles smaller than the openings fall through the screen and are collected. Particles larger than the specified size of the openings are retained and separated from the smaller paricles. In the present invention, such retained particles are further milled to produce smaller particles and then placed over the screen to collect additional particles that can pass through the screen openings.

In a preferred embodiment of this method, granules of divalproex sodium are mixed with silica gel and suspended in ethanol. The granules are then dried in warm air and passed through sizing screens to produce granules smaller than a selected size. The sized granules are then mixed with a coating composition comprising ethycellulose, castor oil, magnesium stearate, acetone and ethanol. The coated drug granules are then sifted through another screen to obtain desired-size coated granules which are then blended together with methylcellulose, hydroxypropylmethylcellulose, calcium phosphate, stearic acid and talc and compressed into tablets.

In another preferred embodiment of this method, terazosin hydrochloride dihydrate and povidone in ethanol are coated on sugar spheres. The terazsosin particles are then mixed with a coating composition comprising ethylcellulose, castor oil, magnesium stearate, ethanol and acetone. The coated drug particles are sifted through a sizing screen and blended together with methylcellulose, hydroxylpropylmethylcellulose, calcium phosphate, stearic acid and talc and compressed into tablets

The following examples will serve to further illustrate the invention.

### EXAMPLE 1

### DIVALPROEX SODIUM TABLETS

### 1A PREPARATION OF GRANULAR DRUG PARTICLES

Divalproex sodium (19.2 kg) was mixed with pharmaceutical-grade silica gel (800 g, Syloid 244) and the mixture was milled in a Fitzmill at medium speed with knives forward through a 2A band, for 5 minutes.

The milled mixture was then placed in a granulation mixer, and ethanol (1.2 kg, 200 proof) was added during mixing at low speed until the mixture was granular (about 3 minutes). The granular mixture was removed and dried in an Aeromatic Fluid Bed Dryer at 45°C to 50°C. The granulated mixture was then sifted through a 12-mesh and then a 24-mesh screen. The material that passed through the 12-mesh screen and that was retained on the 24-mesh screen was collected. The material retained on the 12-mesh screen was re-milled in a Fitzmill at medium speed through a 2A band and then resifted through 12-mesh and 24-mesh screens.

### 1B DRUG GRANULE COATING COMPOSITION

Ethanol (12 liters, 200 proof) and acetone (40 liters) were mixed together. Triethyl citrate (600 g) and ethylcellulose (3600 g) were slowly added to the mixture, and mixing continued to produce a clear solution. Magnesium stearate (3600 g) and blue dye (12 g, FD and C No. 2) were added with mixing. Acetone was then added to bring the volume to 60 liters.

### 1C COMPRESSION ENHANCING COATING COMPOSITION

Ethanol (25 liters, 200 proof) was combined with povidone (300 g) and mixed for about one hour to produce a clear solution. Microcrystalline cellulose (3000 g) was added to the solution with continued mixing. Ethanol (200 proof) was then added to bring the volume to 30 liters.

### 1D COATING OF DRUG GRANULES

The coating composition of EXAMPLE 1B was applied to the drug granules (12-24 mesh) of EXAMPLE 1A at a concentration of 3 liters of coating composition per 1 kg of drug granules. The compression coating composition of EXAMPLE 1C (1.5 liters per kg of particles) was added to the mixture. The particles were dried by fluidizing without spraying for 30 minutes to produce a loss on drying (L.O.D.) of not more than 0.5 percent at 110°C.

The coated particles were collected and sifted through a 10-mesh screen and the coated particles that passed through the screen were collected.

### 1E PREPARATION OF TABLETS

The coated particles (4.2 kg) obtained in EXAMPLE 1D were combined with methylcellulose (250 g, 15 cps), hydroxypropylmethylcellulose (950 g, USP 2910, 100 cps), calcium phosphate (950 g), stearic acid (50 g) and talc (100 g), and mixed in a blender. The blended mixture was then compressed into tablets.

### EXAMPLE 2

### DIVALPROEX SODIUM TABLETS

### 2A PREPARATION OF GRANULAR DRUG PARTICLES

Divalproex sodium (1440 kg) was mixed with pharmaceutical-grade silica gel (60 kg, Syloid 244) and the mixture was milled in a Fitzmill at medium speed with knives forward through a 2A band, for 5 minutes.

The milled mixture was then placed in a granulation mixer with ethanol (100 liters, 200 proof) and mixed at low speed until the mixture was granular. The granular mixture was removed and dried in an Aeromatic Fluid Bed Dryer at 45°C to 50°C to produce a loss on drying (L.O.D.) of not more than 0.5 percent. The granulated mixture was then sifted through a 12-mesh and then a 24-mesh screen. The material that passed through the 12-mesh screen and that was retained on the 24-mesh screen was collected. The material retained on the 12-mesh screen was re-milled in a Fitzmill at medium speed through a 2A band and then resifted through 12-mesh and 24-mesh screens.

### 2B DRUG GRANULE COATING COMPOSITION

Ethanol (150 liters, 200 proof) and acetone (450 liters) were mixed together. Castor oil (7.5 kg) and ethylcellulose (45 kg) were slowly added to the mixture, and mixing continued to produce a clear solution. Magnesium stearate (45 kg) and blue dye (300 g, FD and C No. 2) and yellow dye (56.25 g, D and C No. 10) were added with mixing. Acetone was then added to bring the volume to 750 liters.

### 2C COMPRESSION ENHANCING COATING COMPOSITION

Ethanol (280 liters, 200 proof) was combined with povidone (7.6 kg) and mixed for about one hour to produce a clear solution. Microcrystalline cellulose (76 kg) was added to the solution with continued mixing. Ethanol (200 proof) was then added to bring the volume to 380 liters.

### 2D COATING OF DRUG GRANULES

The coating composition of EXAMPLE 2B was applied to the drug granules (12-24 mesh) of EXAMPLE 2A at a concentration of 3 liters of coating composition per 1 kg of drug granules. (Inlet Air Temp.: 50°C; Relative Humidity: 0%; Chamber Pressure: -10 mm H₂O; Atomization Air Pressure; 65 PSIG; Process Air Flow Rate: 4000 SCMH; Solution Flow Rate: 310 ml/minute/nozzle). The compression coating composition of EXAMPLE 2C (0.75 liters per kg of particles) was added to the mixture. (Inlet Air Temp.: 48-50°C; Relative Humidity: 0%; Chamber Pressure: -10 mm H₂O; Atomization Air Pressure: 40 PSIG; Process Air Flow Rate: 6000 SCMH; Solution Flow Rate: 350 ml/minute/nozzle) . The particles were dried by fluidizing without spraying for 20 minutes to produce a loss on drying (L.O.D.) of not more than 0.5 percent at 110°C.

The coated particles were collected and sifted through a 10-mesh screen and the coated particles that passed through the screen were collected.

### 2E PREPARATION OF TABLETS

The coated particles (98.04 kg) obtained in EXAMPLE 2D were combined with methylcellulose (6.75 kg, 15 cps), hydroxypropylmethylcellulose (22.5 kg, USP 2208, 100 cps), calcium phosphate (22.5 kg), stearic acid (1.125 kg) and talc (3.375 kg), and mixed in a blender. The blended mixture was then compressed into tablets.

### EXAMPLE 3

### BIOAVAILBILITY STUDY

Drug tablets were administered to 15 adult males under fasting and non-fasting conditions.

Two formulations of divalproex sodium were utilized to determine bioavailability data.

Formulation A was the controlled-release dosage form of the present invention containing 500 mg valproic acid equivalent per tablet and prepared according to the general procedure of Example 2.

Formulation B was an enteric coated tablet (Depakote®) containing 250 mg valproic acid equivalent per tablet.

A single dose of 1000 mg valproic acid equivalent (two tablets of Formulation A, or four tablets of Formulation B) was administered to each subject. Each subject was a healthy adult male between 18 and 40 years of age. Formulation A was administered under fasting and non-fasting conditions.

The subjects were housed and supervised for four and one-half days in each study period, from a minimum of 12 hours prior to administration of the drug dose through the 72-hour blood collection.

The subjects abstained from all food and beverage except for scheduled meals and water during the study period. Under non-fasting conditions, the subjects were served a meal one-half hour prior to administering a dose of Formulation A. Under fasting conditions, the subjects were served a meal 2 hours after administration of doses of Formulations A and B, respectively. All subjects received a meal 6 hours after drug administration and, thereafter, at 11 hours, 24.5 hours, 28 hours, and 34 hours after drug administration. Seven-milliliter blood samples were collected in heparinized tubes from each subject prior to drug administration (0 hour) and at 1, 2, 3, 4, 6, 8, 10, 12, 15, 18, 24, 30, 36, 48, 60 and 72 hours after drug administration, and stored at 0°C until separation into plasma (within 3 hours after collection and plasma was stored at -10°C).

The results obtained are shown in TABLE I.

**TABLE I**

| Plasma Valproic Acid Concentration (µg/ml) | | | | | | |
|---|---|---|---|---|---|---|
| Time (hours) | Formulation A (fasting) | | Formulation A (non-fasting) | | Formulation B (fasting) | |
| 0 | 0.0 | (0.1) | 0.0 | (0.0) | 0.0 | (0.1) |
| 1 | 3.9 | (1.2) | 1.4 | (0.9) | 1.4 | (2.6) |
| 2 | 12.3 | (3.1) | 12.8 | (21.9) | 47.2 | (36.9) |
| 3 | 21.3 | (4.7) | 20.6 | (23.8) | 72.0 | (31.2) |
| 4 | 36.1 | (23.1) | 28.4 | (19.0) | 60.5 | (30.5) |
| 6 | 35.2 | (6.7) | 43.1 | (11.2) | 59.4 | (19.5) |
| 8 | 47.1 | (8.2) | 49.4 | (7.7) | 58.7 | (7.4) |
| 10 | 54.3 | (7.4) | 55.7 | (9.9) | 52.2 | (6.7) |
| 12 | 60.1 | (9.0) | 58.0 | (7.7) | 50.1 | (13.0) |
| 15 | 52.0 | (7.9) | 51.0 | (7.0) | 41.5 | (10.6) |
| 18 | 44.8 | (6.9) | 42.9 | (7.8) | 34.3 | (9.6) |
| 24 | 34.3 | (6.3) | 33.9 | (5.8) | 27.1 | (8.9) |
| 30 | 23.4 | (6.4) | 22.6 | (4.7) | 18.3 | (5.7) |
| 36 | 17.5 | (4.4) | 16.7 | (4.3) | 13.6 | (4.6) |
| 48 | 10.0 | (3.5) | 9.7 | (3.4) | 8.4 | (3.4) |
| 60 | 5.6 | (2.3) | 5.5 | (2.2) | 4.8 | (2.4) |
| 72 | 3.7 | (1.8) | 3.5 | (1.8) | 3.1 | (1.9) |
| Tₘₐₓ | 10.9 | (3.4) | 11.0 | (2.7) | 4.3 | (3.3) |
| Cₘₐₓ | 65.1 | (11.9) | 63.7 | (12.6) | 87.0 | (9.5) |
| AUC | 1585.0 | (278.8) | 1557.6 | (244.0) | 1527.9 | (264.6) |
| Tₘₐₓ = Time of peak concentration (hours). Cₘₐₓ = Peak concentration (µg/ml). AUC = Area under Plasma Concentration - Time Curve, 0 to 72 hours (µg x hr/ml). The values in parentheses represent the standard deviation. | | | | | | |

The results show that the controlled-release dosage form of the present invention had a lower Cₘₐₓ in plasma in both fasting and non-fasting subjects than the enteric coated tablets, and maintained a higher plasma concentration of valproic acid over time.

### EXAMPLE 4

### TERAZOSIN HYDROCHLORIDE DIHYDRATE TABLETS

### 4A PREPARATION OF DRUG PARTICLES

Terazosin hydrochloride dihydrate (48.0 kg) was mixed with povidone (16.0 kg) and ethanol (300 liters, 200 proof). After mixing, ethanol (200 proof) was added to bring the final volume to 400 liters.

200 kg of sugar spheres (40-50 mesh) were coated with the above-mentioned solution of terazosin. (Inlet Air Temp.: 52°C; Relative Humidity: 15%; Chamber Pressure: -10 mm H₂O; Atomization Air Pressure; 65 PSIG; Process Air Flow Rate: 3000-3500 SCMH; Solution Flow Rate: 200-215 ml/minute/nozzle).

### 4B DRUG PARTICLE COATING COMPOSITION

Ethanol (100 liters, 200 proof) and acetone (300 liters) were mixed together. Ethylcelluose (30 kg) and castor oil (5.0 kg) were slowly added to the mixture and mixing continued to obtain a solution. Magnesium stearate (30kg) was added with mixing and acetone was then added to bring the final volume to 500 liters.

### 4C COMPRESSION ENHANCING COATING COMPOSITION

Ethanol (140 liters, 200 proof) was combined with povidone (4.0 kg) and mixed for about 15 minutes to obtain a clear solution. Microcrystalline cellulose (40.0 kg) was added to the solution with continued mixing. Ethanol (200 proof) was added to bring the final volume to 200 liters.

### 4D COATING OF DRUG PARTICLES

The drug particles of Example 4A were coated with the particle coating composition of Example 4B (2.5 liters per kg of nonpareils). (Inlet Air Temp.: 48°C; Relative Humidity: 15%; Chamber Pressure: -10 mm H₂O; Atomization Air Pressure; 65 PSIG; Process Air Flow Rate: 3600 SCMH; Solution Flow Rate: 200-220 ml/minute/nozzle). The coated particles were dried by fluidizing without spraying for 20 minutes. The dried coated particles were sifted through a 20/60 mesh sifter.

The sifted partices were then coated with the compression enhancing coating composition of Example 4C (1.0 liters per kg of nonpareils). (Inlet Air Temp.: 50°C; Relative Humidity: 15%; Chamber Pressure: -10 mm H₂O; Atomization Air Pressure; 40 PSIG; Process Air Flow Rate: 3600 SCMH; Solution Flow Rate: 180 ml/minute/nozzle). The coated particles were dried by fluidizing without spraying for 20 minutes to produce a loss on drying (L.O.D.) of not more than 1.5%. The dried coated particles were sifted through a 20/60 mesh sifter. The particles that passed through the 20 mesh screen but that did not pass through the 60 mesh screen were collected.

### 4E PLACEBO PARTICLE COATING COMPOSITION

Ethanol (200 liters, 200 proof) and acetone (600 liters) were mixed together. Ethylcellulose (60 kg) and castor oil (10 kg) were slowly added to the mixture and mixing continued to obtain a solution. Magnesium stearate (60 kg) was added with mixing and acetone was added to bring the final volume to 1000 liters.

### 4F COMPRESSION ENHANCING COATING COMPOSITION

Ethanol (300 liters, 200 proof) was combined with povidone (8.0 kg) and mixed for about 15 minutes to obtain a clear solution. Microcrystalline cellulose (80 kg) was added to the solution wilth continued mixing. Ethanol (200 proof) was added to bring the final volume to 400 liters.

### 4G COATING OF PLACEBO PARTICLES

300 kg of sugar spheres (40-50 mesh) were coated with the particle coating composition of Example 4E (500 liters). (Inlet Air Temp.: 48-50°C; Relative Humidity: 30%; Chamber Pressure: -10 mm H₂O; Atomization Air Pressure; 65 PSIG; Process Air Flow Rate: 3500-4000 SCMH; Solution Flow Rate: 310 ml/minute/nozzle). The coated particles were dried by fluidizing without spraying for 15 minutes. The dried coated particles were sifted through a 20/60 mesh sifter.

The sifted particles were then coated with 200 litres of the compression enhancing coating of Example 4F. (Inlet Air Temp.: 50°C; Relative Humidity: 30%; Chamber Pressure: -10mm H_{²}O; Atomization Air Pressure; 40 PSIG; Process Air Flow Rate: 4500 SCMH; Solution Flow Rate: 200 ml/minute/nozzle). The coated particles were dried by fluidizing without spraying for 20 minutes to produce a loss on drying (L.O.D.) of not more than 1.5%. The dried coated particles were sifted through a 20/60 mesh sifter. The particles that passed through the 20 mesh screen but did not pass through the 60 mesh screen were collected.

### 4H PREPARATION OF TABLETS

Methylcellulose (0.875 kg), hydroxypropyl methylcellulose (3.675 kg), calcium phosphate (11.9 kg), stearic acid (0.4 kg) and talc (0.65 kg) were mixed using a Fitzmill at high speed with knives forward through a 1-A band.

The mixture was charged into a blender along with the coated terazosin particles of Example 4D (2.066 kg) and the coated placebo particles of Example 4G (15.434 kg) and blended for 10 minutes. The blended mixture was then compressed into tablets.

## Claims (Claims for the following Contracting State(s): AT, BE, CH, DE, FR, GB, IT, LI, LU, NL, SE)

1. A sustained-release drug dosage form comprising (1) granules of a drug coated with a sustained-release coating composition comprising from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent and (2) a slow release matrix comprising at least one viscosity agent, wherein the drug is selected from the group consisting of is divalproex sodium, valproic acid or amides or esters or salts thereof; or terazosin or salts, hydrates or derivatives thereof.

2. The drug dosage form of Claim 1 wherein the sustained-release coated drug particle comprises:
from 62 to 86 weight percent divalproex sodium:
from 2.6 to 3.7 weight percent silica gel;
from 2.6 to 11.6 weight percent ethylcellulose;
from 0.4 to 1.9 weight percent castor oil;
from 2.6 to 11.6 weight percent magnesium stearate:
from 0 to 0.09 weight percent dye;
from 0.4 to 1.0 weight percent povidone; and
from 4.5 to 9.7 weight percent microcrystalline cellulose.

3. The drug dosage form of Claim 1 wherein the sustained-release coated drug particle comprises:
about 12.9 weight percent terazosin monohydrochloride dihydrate;
about 5.5 weight percent povidone;
about 53.6 weight percent sugar spheres;
about 8.0 weight percent ethylcellulose;
about 1.3 weight percent castor oil;
about 8.0 weight percent magnesium stearate; and
about 10.7 weight percent microcrystalline cellulose.

4. A method for the preparation of a sustained-release drug dosage form of claim 1 comprising:
a) admixing said drug with a detackifying agent;
b) adding a solvent to said admixture with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer and a detackifying agent to produce coated granules;
f) mixing said coated granules with at least one viscosity agent; and
g) forming said mixture produced in step (f) into drug dosage units.

5. A method according to Claim 4 for the preparation of a sustained-release drug dosage form comprising:
a) admixing said drug with silica gel;
b) adding a solvent to said admixture, with constant mixing, to produce drug granules;
c) drying said drug granules;
d) sifting and sizing said granules to obtain desired size granules;
e) mixing said desired sized granules with a coating composition said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel to produce coated granules;
f) mixing said coated granules with at least one viscosity agent selected from the group consisting of methylcellulose, hydroxypropylmethylcellulose, povidone and hydroxypropyl-cellulose; and
g) forming said mixture produced in step (f) into drug dosage units.

6. A method for the preparation of a sustained-release drug dosage form comprising:
a) coating sugar spheres with a solution of the drug to produce drug granules;
b) mixing said granules with a coating composition, said coating composition comprising an ethylcellulose or a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent; and
f) forming said mixture produced in step (e) into drug dosage units,
wherein said drug is selected from the group consisting of divalproex sodium, valproic acid or derivatives or salts thereof; or terazosin, its salts, hydrates or derivatives thereof.

7. A sustained-release drug dosage form comprising (1) granules of a drug coated with a sustained-release coating composition comprising from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent and (2) a slow release matrix comprising at least one viscosity agent, and calcium phosphate.

8. A sustained-release drug dosage form comprising (1) granules of a drug coated with a sustained-release coating composition comprising from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent and (2) a slow release matrix comprising at least one viscosity agent selected from the group consisting of methyl cellulose, hydroxypropylmethylcellulose, povidone and hydroxypropyl-cellulose and further comprising calcium phosphate.

9. The drug dosage form of Claim 7 or 8, wherein said plasticizer is selected from the group consisting of castor oil, propylene glycol, polyethylene glycol, acetyl triethyl citrate, acetyl tributyl citrate, triethyl citrate and tributyl citrate and said detackifying agent is selected from the group consisting of magnesium stearate, talc, titanium dioxide and silica gel and said viscosity agent is selected from the group consisting of methylcellulose, hydroxypropylcellulose, povidone, and hydroxypropylmethylcellulose.

10. The drug dosage form of Claim 7 or 8 comprising:
from 2.0 to 80 weight percent sustained-release coated drug granules;
up to 30 weight percent calcium phosphate;
from 2 to 30 weight percent of a first viscosity agent and from 0 to 30 weight percent of a second viscosity agent wherein said first and second viscosity agents are independently selected from the group consisting of methylcellulose, hydroxypropylmethylcellulose, povidone and hydroxypropylcellulose.

11. The drug dosage form of Claim 7 or 8 comprising:
from 63 to 65 weight percent of sustained-release coated divalproex sodium granules;
from 3.5 to 4.5 weight percent of methylcellulose;
from 14.2 to 14.6 weight percent of hydroxypropylmethylcellulose;
from 14.2 to 14.6 weight percent of calcium phosphate;
from 0.7 to 0.8 weight percent of stearic acid; and
from 1.5 to 2.2 weight percent of talc.

12. The drug dosage form of Claim 7 or 8 comprising:
from 2.6 to 26.4 weight percent of sustained-release coated terazosin monohydrochloride dihydrate granules;
about 2.5 weight percent of methylcellulose;
about 10.5 weight percent of hydroxypropylmethylcellulose;
about 34.0 weight percent of calcium phosphate;
about 1.1 weight percent of stearic acid; and
about 1.9 weight percent of talc.

13. A method for the preparation of a sustained-release drug dosage form of claim 7 comprising:
a) admixing a drug with a detackifying agent;
b) adding a solvent to said admixture with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer and a detackifying agent to produce coated granules;
f) mixing said coated granules with at least one viscosity agent and calcium phosphate and
g) forming said mixture produced in step (f) into drug dosage units.

14. A method for the preparation of a sustained-release drug dosage form of claim 8 comprising:
a) admixing a drug with a detackifying agent;
b) adding a solvent to said admixture with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer and a detackifying agent to produce coated granules;
f) mixing said coated granules with said at least one viscosity agent; and
g) forming said mixture produced in step (f) into drug dosage units,
wherein the composition of step (f) further comprises calcium phosphate.

15. A method according to Claim 13 or 14 wherein said detackifying agent in step (a) is silica.

16. A method according to Claim 13 or 14 wherein said plasticizer of step (e) is selected from the group consisting of castor oil, propylene glycol, polyethylene glycol, acetyl triethyl citrate, acetyl tributyl citrate, triethyl citrate and tributyl citrate; and wherein said detackifying agent of step (e) is selected from the group consisting of magnesium stearate, talc, titanium dioxide and silica gel.

17. A method according to Claim 13 or 14 for the preparation of a sustained-release drug dosage form comprising:
a) admixing a drug with silica gel;
b) adding a solvent to said admixture, with constant mixing, to produce drug granules;
c) drying said drug granules;
d) sifting and sizing said granules to obtain desired size granules;
e) mixing said desired sized granules with a coating composition said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel to produce coated granules;
f) mixing said coated granules with at least one viscosity agent selected from the group consisting of methylcellulose, hydroxypropylmethylcellulose, povidone and hydroxypropyl-cellulose; and
g) forming said mixture produced in step (f) into drug dosage units.

18. A method for the preparation of a sustained-release drug dosage form comprising:
a) coating sugar spheres with a solution of the drug to produce drug granules;
b) mixing said granules with a coating composition, said coating composition comprising an ethylcellulose or a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent; and
f) forming said mixture produced in step (e) into drug dosage units, wherein said viscosity agent is selected from the group consisting of methyl cellulose, hydroxypropylmethylcellulose, povidone and hydroxypropylcellulose and the composition of step (e) further comprises calcium phosphate.

19. A method according to Claim 18 wherein said coating composition comprises from 2 to 20 weight percent (g/ml x 100) of an ethylcellulose or a methacrylic methylester, from 0.1 to 5.O weight percent (g/mix 100) of a plasticizer and from O.5 to 20 weight percent (g/ml x 100) of a detackifying agent.

20. A method according to Claim 19 wherein said plasticizer of step (b) is selected from the group consisting of castor oil, propylene glycol, polyethylene glycol, acetyl triethyl citrate, acetyl tributyl citrate, triethyl citrate and tributyl citrate; and wherein said detackifying agent of step (b) is selected from the group consisting of magnesium stearate, talc, titanium dioxide and silica gel.

## Claims (Claims for the following Contracting State(s): DK)

1. A sustained-release coated drug comprising granules of said drug coated with a coating composition comprising an ethylcellulose or a methacrylic methylester, a plasticizer and a detackifying agent, wherein the coating composition comprises ethylcellulose, castor oil as said plasticizer and magnesium stearate as said detackifying agent and wherein said drug is divalproex sodium or terazosin monohydrochloride dihydrate.

2. A sustained-release coated drug particle of claim 1 comprising:
from 62 to 86 weight percent divalproex sodium;
from 2.6 to 3.7 weight percent silica gel;
from 2.6 to 11.6 weight percent ethylcellulose.
from 0.4 to 1.9 weight percent castor oil;
from 2.6 to 11.6 weight percent magnesium stearate;
from 0 to 0.09 weight percent dye;
from 0.4 to 1.0 weight percent povidone; and
from 4.5 to 9.7 weight percent microcrystalline cellulose; or
about 12.9 weight percent terazosin monohydrochloride dihydrate;
about 5.5 weight percent povidone;
about 53.6 weight percent sugar spheres;
about 8.0 weight percent ethylcellulose;
about 1.3 weight percent castor oil;
about 8.0 weight percent magnesium stearate; and
about 10.7 weight percent microcrystalline cellulose.

3. An orally administrable sustained-release drug dosage unit comprising a composition of at least one viscosity agent together with drug granules coated with a sustained-release coating comprising an ethylcellulose or a methacrylic methylester, a plasticizer and a detackifying agent wherein said drug is selected from the group consisting of divalproex sodium, valproic acid or derivatives or salts thereof; and terazosin or salts or hydrates or derivatives thereof.

4. A method for the preparation of a sustained-release drug dosage of claim 3 comprising:
a) admixing said drug with a detackifying agent;
b) adding a solvent to said admixture, with constant mixing, to produce drug granules;
c) drying said drug granules,
d) sifting and sizing said granules to obtain desired size granules;
e) mixing said desired sized granules with a coating composition, said coating composition comprising an ethylcellulose or a methacrylic methylester, a plasticizer and a detackifying agent to produce coated granules;
f) mixing said coated granules with at least one viscosity agent; and
g) forming said mixture produced in step (i) into drug dosage units.

5. A method according to claim 4 for the preparation of a sustained-release drug dosage comprising:
a) admixing said drug with silica gel;
b) adding a solvent to said admixture, with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel to produce coated granules;
f) mixing said coated granules with at least one viscosity agent selected from the group consisting of methylcellulose, hydroxypropylmethylcellulose, povidone and hydroxypropyl-cellulose; and
g) forming said mixture produced in step (f) into drug dosage units.

6. A method for the preparation of a sustained-release drug dosage of claim 3 comprising:
a) coating sugar spheres with a solution of the drug to produce drug granules;
b) mixing said granules with a coating composition, said coating composition comprising an ethylcellulose of a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent; and
f) forming said mixture produced in step (e) into drug dosage units.

7. An orally administrable sustained-release drug dosage unit comprising a composition of at least one viscosity agent together with drug granules coated with a sustained-release coating comprising an ethylcellulose or a methacrylic methylester, a plasticizer and a detackifying agent, wherein the composition further comprises calcium phosphate.

8. An orally administrable sustained-release drug dosage unit comprising a composition of at least one viscosity agent together with drug granules coated with a sustained-release coating comprising an ethylcellulose or a methacrylic methylester, a plasticizer and a detackifying agent, wherein said viscosity agent is selected from the group consisting of methylcellulose, hydroxypropylmethylcellulose, povidone and hydroxypropylcellulose and further comprising calcium phosphate in said composition.

9. The dosage unit of Claim 7 or 8 comprising:
from 2.0 to 80 weight percent sustained-release coated drug granules;
up to 30 weight percent calcium phosphate;
from 2 to 30 weight percent of a first viscosity agent and from 0 to 30 weight percent of a second viscosity agent wherein said first and second viscosity agents are independently selected from the group consisting of methylcellulose, hydroxypropylmethylcellulose, povidone and hydroxypropylcellulose.

10. The dosage unit of Claim 7 or 8 wherein said drug is selected from the group consisting of divalproex sodium, valproic acid or derivatives or salts thereof; and terazosin or salts or hydrates or derivatives thereof.

11. The dosage unit of Claim 7 or 8 comprising:
from 63 to 65 weight percent of sustained-release coated divalproex sodium granules;
from 3.5 to 4.5 weight percent of methylcellulose;
from 14.2 to 14.6 weight percent of hydroxypropylmethylcellulose;
from 14.2 to 14.6 weight percent of calcium phosphate;
from 0.7 to 0.8 weight percent of stearic acid; and
from 1.5 to 2.2 weight percent of talc; or
from 2.6 to 26.4 weight percent of sustained-release coated terazosin monohydrochloride dihydrate granules;
about 2.5 weight percent of methylcellulose;
about 10.5 weight percent of hydroxypropylmethylcellulose;
about 34.0 weight percent of calcium phosphate;
about 1.1 weight percent of stearic acid; and
about 1.9 weight percent of talc.

12. A method for the preparation of a sustained-release drug dosage of claim 7 or 8 comprising:
a) admixing a drug with a detackifying agent;
b) adding a solvent to said admixture, with constant mixing, to produce drug granules;
c) drying said drug granules,
d) sifting and sizing said granules to obtain desired size granules;
e) mixing said desired sized granules with a coating composition, said coating composition comprising an ethylcellulose or a methacrylic methylester, a plasticizer and a detackifying agent to produce coated granules;
f) mixing said coated granules with at least one viscosity agent; and
g) forming said mixture produced in step (f) into drug dosage units, wherein said viscosity agent is selected from the group consisting of methyl cellulose, hydroxypropylmethylcellulose, povidone and hydroxypropylcellulose and the composition of step (f) further comprises calcium phosphate.

13. A method according to Claim 12 wherein said detackifying agent in step (a) is silica.

14. A method according to Claim 12 wherein said coating composition comprises from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent.

15. A method according to claim 12 wherein said plasticizer of step (e) is selected from the group consisting of castor oil, propylene glycol, polyethylene glycol, acetyl triethyl citrate, acetyl tributyl citrate, triethyl citrate and tributyl citrate; and wherein said detackifying agent of step (e) is selected from the group consisting of magnesium stearate, talc, titanium dioxide and silica gel.

16. A method according to claim 12 for the preparation of a sustained-release drug dosage comprising:
a) admixing said drug with silica gel;
b) adding a solvent to said admixture, with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel to produce coated granules;
f) mixing said coated granules with at least one viscosity agent selected from the group consisting of methylcellulose, hydroxypropylmethylcellulose, povidone and hydroxypropyl-cellulose and with calcium phosphate; and
g) forming said mixture produced in step (f) into drug dosage units.

17. A method according to Claim 12 or 16 wherein said drug is selected from the group consisting of divalproex sodium, valproic acid or derivatives or salts thereof; or terazosin, its salts, hydrates or derivatives thereof.

18. A method for the preparation of a sustained-release drug dosage of claim 7 or 8 comprising:
a) coating sugar spheres with a solution of the drug to produce drug granules;
b) mixing said granules with a coating composition, said coating composition comprising an ethylcellulose or a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent and with calcium phosphate; and
f) forming said mixture produced in step (e) into drug dosage units.

19. A method according to Claim 18 wherein said coating composition comprises from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent.

20. A method according to Claim 18 wherein said plasticizer of step (b) is selected from the group consisting of castor oil, propylene glycol, polyethylene glycol, acetyl triethyl citrate, acetyl tributyl citrate, triethyl citrate and tributyl citrate; and wherein said detackifying agent of step (b) is selected from the group consisting of magnesium stearate, talc, titanium dioxide and silica gel.

21. A method according to Claim 18 wherein said drug is selected from the group consisting of divalproex sodium, valproic acid or derivatives or salts thereof; or terazosin, its salts, hydrates or derivatives thereof.

## Claims (Claims for the following Contracting State(s): ES, GR)

1. A method for the preparation of a sustained-release drug dosage form comprising
(1) granules of a drug coated with a sustained-release coating composition comprising from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent and (2) a slow release matrix comprising at least one viscosity agent, said method comprising:
a) admixing a drug with a detackifying agent;
b) adding a solvent to said admixture, with constant mixing, to produce drug granules:
c) drying said drug granules;
d) sifting and sizing said granules to obtain desired size granules;
e) mixing said desired sized granules with a said coating composition to produce coated granules;
f) mixing said coated granules with at least one viscosity agent; and
g) forming said mixture produced in step (f) into drug dosage units, wherein said drug is selected from the group consisting of divalproex sodium, valproic acid or derivatives or salts thereof; or terazosin, its salts, hydrates or derivatives thereof.

2. A method according to Claim 1 for the preparation of a sustained-release drug dosage comprising:
a) admixing said drug with silica gel;
b) adding a solvent to said admixture, with constant mixing, to produce drug granules;
c) drying said drug granules;
d) sifting and sizing said granules to obtain desired size granules;
e) mixing said desired sized granules with a coating composition, said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel to produce coated granules;
f) mixing said coated granules with at least one viscosity agent selected from the group consisting of methylcellulose, hydroxypropylmethylcellulose, povidone and hydroxypropyl-cellulose; and
g) forming said mixture produced in step (f) into drug dosage units.

3. A method according to Claim 1 for the preparation of a drug dosage form wherein the sustained-release coated drug particle comprises:
from 62 to 86 weight percent divalproex sodium:
from 2.6 to 3.7 weight percent silica gel;
from 2.6 to 11.6 weight percent ethylcellulose
from 0.4 to l.9 weight percent castor oil;
from 2.6 to 11.6 weight percent magnesium stearate;
from O to 0.09 weight percent dye;
from 0.4 to 1.0 weight percent povidone; and
from 4.5 to 9.7 weight percent microcrystalline cellulose.

4. A method according to Claim 1 for the preparation of a drug dosage form wherein the sustained-release coated drug particle comprises:
about 12.9 weight percent terazosin monohydrochloride dihydrate;
about 5.5 weight percent povidone;
about 53.6 weight percent sugar spheres;
about 8.0 weight percent ethylcellulose;
about 1.3 weight percent castor oil;
about 8.0 weight percent magnesium stearate; and
about 10.7 weight percent microcrystalline cellulose.

5. A method for the preparation of a sustained-release drug dosage form comprising:
a) coating sugar spheres with a solution of the drug to produce drug granules;
b) mixing said granules with a coating composition, said coating composition comprising an ethylcellulose or a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent; and
f) forming said mixture produced in step (e) into drug dosage units, wherein said drug is selected from the group consisting of divalproex sodium, valproic acid or derivatives or salts thereof; or terazosin, its salts, hydrates or derivatives thereof.

6. A method for the preparation of a sustained-release drug dosage form comprising
(1) granules of a drug coated with a sustained-release coating composition comprising from 2 to 20 weight percent (g/ml x 100) of an ethylcellulose or a methacrylic methylester, from 0.1 to 5.0 weight percent (g/ml x 100) of a plasticizer and from 0.5 to 20 weight percent (g/ml x 100) of a detackifying agent and (2) a slow release matrix comprising at least one viscosity agent, said method comprising:
a) admixing a drug with a detackifying agent;
b) adding a solvent to said admixture, with constant mixing, to produce drug granules;
c) drying said drug granules;
d) sifting and sizing said granules to obtain desired size granules;
e) mixing said desired sized granules with a said coating composition to produce coated granules;
f) mixing said coated granules with at least one viscosity agent; and
g) forming said mixture produced in step (f) into drug dosage units, wherein said viscosity agent is selected from the group consisting of methyl cellulose, hydroxypropylmethylcellulose, povidone and hydroxypropylcellulose and the composition of step (f) further comprises calcium phosphate.

7. A method according to Claim 6 wherein said detackifying agent in step (a) is silica.

8. A method according to Claim 6 wherein said plasticizer of step (e) is selected from the group consisting of castor oil, propylene glycol, polyethylene glycol, acetyl triethyl citrate, acetyl tributyl citrate, triethyl citrate and tributyl citrate; and wherein said detackifying agent of step (e) is selected from the group consisting of magnesium stearate, talc, titanium dioxide and silica gel.

9. A method according to Claim 6 for the preparation of a sustained-release drug dosage comprising:
a) admixing a drug with silica gel;
b) adding a solvent to said admixture, with constant mixing, to produce drug granules;
c) drying said drug granules;
d) sifting and sizing said granules to obtain desired size granules;
e) mixing said desired sized granules with a said coating composition, said coating composition comprising an ethylcellulose or a methacrylic methylester, a plasticizer selected from the group consisting of castor oil, propylene glycol, polyethylene glycol, acetyl triethyl citrate, acetyl tributyl citrate, triethyl citrate and tributyl citrate and a detackifying agent selected from the group consisting of magnesium stearate, talc, titanium dioxide and silica gel, to produce coated granules;
f) mixing said coated granules with at least one viscosity agent selected from the group consisting of methylcellulose, hydroxypropylmethylcellulose, povidone and hydroxypropyl-cellulose and with calcium phosphate; and
g) forming said mixture produced in step (f) into drug dosage units.

10. A method according to Claim 6 for the preparation of a drug dosage form comprising: from 2.0 to 80 weight percent sustained-release coated drug granules; up to 30 weight percent calcium phosphate; from 2 to 30 weight percent of a first viscosity agent and from 0 to 30 weight percent of a second viscosity agent wherein said first and second viscosity agents are independently selected from the group consisting of methylcellulose, hydroxypropylmethylcellulose, povidone and hydroxypropylcellulose.

11. A method according to Claim 6 for the preparation of a drug dosage form comprising:
from 63 to 65 weight percent of sustained-release coated divalproex sodium granules;
from 3.5 to 4.5 weight percent of methylcellulose;
from 14.2 to 14.6 weight percent of hydroxypropylmethylcellulose;
from 14.2 to 14.6 weight percent of calcium phosphate;
from 0.7 to 0.8 weight percent of stearic acid; and
from 1.5 to 2.2 weight percent of talc.

12. A method according to Claim 6 for the preparation of a drug dosage form comprising:
from 2.6 to 26.4 weight percent of sustained-release coated terazosin monohydrochloride dihydrate granules;
about 2.5 weight percent of methylcellulose;
about 10.5 weight percent of hydroxypropylmethylcellulose;
about 34.0 weight percent of calcium phosphate;
about 1.1 weight percent of stearic acid; and
about 1.9 weight percent of talc.

13. A method for the preparation of a sustained-release drug dosage form comprising:
a) coating sugar spheres with a solution of the drug to produce drug granules;
b) mixing said granules with a coating composition, said coating composition comprising an ethylcellulose or a methacrylic methylester, a plasticizer and a detackifying agent to produce coated granules;
c) drying said coated granules;
d) sifting and sizing said coated granules to obtain desired size coated granules;
e) mixing said desired size coated granules with at least one viscosity agent; and
f) forming said mixture produced in step (e) into drug dosage units, wherein said viscosity agent is selected from the group consisting of methylcellulose, hydroxypropylmethylcellulose, povidone and hydroxypropyl-cellulose and the composition of step (e) further comprises calcium phosphate.

14. A method according to Claim 13 wherein said coating composition comprises from 2 to 20 weight percent (g/ml x 100) of an ethylcellulose or a methacrylic methylester, from 0.1 to 5.O weight percent (g/ml x 100) of a plasticizer and from 0.5 to 20 weight percent (g/ml x 100) of a detackifying agent.

15. A method according to Claim 14 wherein said plasticizer of step (b) is selected from the group consisting of castor oil, propylene glycol, polyethylene glycol, acetyl triethyl citrate, acetyl tributyl citrate, triethyl citrate and tributyl citrate; and wherein said detackifying agent of step (b) is selected from the group consisting of magnesium stearate, talc, titanium dioxide and silica gel.

16. A method according to Claim 13 wherein said drug is selected from the group consisting of divalproex sodium, valproic acid or derivatives or salts thereof; or terazosin, its salts, hydrates or derivatives thereof.

## Patentansprüche (Patentansprüche für folgende(n) Vertragsstaat(en): AT, BE, CH, DE, FR, GB, IT, LI, LU, NL, SE)

1. Dosierform mit verzögerter Arzneimittelfreisetzung, die folgendes umfaßt: (1) Granalien eines Arzneimittels, die mit einer Hüllzusammensetzung zur verzögerten Freisetzung, welche 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält, überzogen sind, und (2) eine Matrix mit langsamer Freisetzung, die mindestens ein Viskositätsmittel enthält, wobei das Arzneimittel gewählt ist aus der Gruppe bestehend aus Divalproex-Natrium, Valproinsäure oder Amide oder Ester oder Salze davon; oder Terazosin oder Salze, Hydrate oder Derivate davon.

2. Arzneimitteldosierform nach Anspruch 1, wobei der Arzneistoffpartikel mit einem Überzug zur verzögerten Freisetzung folgendes umfaßt:
von 62 bis 86 Gewichtsprozent Divalproex-Natrium:
von 2,6 bis 3,7 Gewichtsprozent Silicagel;
von 2,6 bis 11,6 Gewichtsprozent Ethylcellulose;
von 0,4 bis 11,9 Gewichtsprozent Rizinusöl;
von 2,6 bis 11,6 Gewichtsprozent Magnesiumstearat:
von 0 bis 0,09 Gewichtsprozent Farbstoff;
von 0,4 bis 1,0 Gewichtsprozent Povidon; und
von 4,5 bis 9,7 Gewichtsprozent mikrokristalline Cellulose.

3. Arzneimitteldosierform nach Anspruch 1, wobei der Arzneistoffpartikel mit einem Überzug zur verzögerten Freisetzung folgendes umfaßt:
etwa 12,9 Gewichtsprozent Terazosinmonohydrochloriddihydrat;
etwa 5,5 Gewichtsprozent Povidon;
etwa 53,6 Gewichtsprozent Zuckerpellets;
etwa 8,0 Gewichtsprozent Ethylcellulose;
etwa 1,3 Gewichtsprozent Rizinusöl;
etwa 8,0 Gewichtsprozent Magnesiumstearat; und
etwa 10,7 Gewichtsprozent mikrokristallifle Cellulose.

4. Verfahren zur Herstellung einer Dosierform mit verzögerter Arzneimittelfreisetzung nach Anspruch 1, das folgende Schritte umfaßt:
a) Beimischen eines Trennmittels zum Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten.

5. Verfahren nach Anspruch 4 zur Herstellung einer Dosierform mit verzögerter Arzneimittelfreisetzung, das folgende Schritte umfaßt:
a) Beimischen von Silicagel zum Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Vermischen der Granalien gewünschter Größe mit einer Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel enthält, das aus der Gruppe bestehend aus Magnesiumstearat, Talkum, Titandioxid und Silicagel gewählt ist, zur Herstellung überzogener Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist; und
g) Formen der Mischung, die in Schritt (f) hergestellt wurde, zu Arzneimitteldosiereinheiten.

6. Verfahren zur Herstellung einer Dosierform mit verzögerter Arzneimittelfreisetzung, das folgendes umfaßt:
a) Überziehen von Zuckerpellets mit einer Lösung des Arzneimittels zur Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel; und
f) Formen der Mischung, die nach Schritt (e) hergestellt wurde, zu Arzneimitteldosiereinheiten, wobei das Arzneimittel aus einer Gruppe gewählt ist bestehend aus Divalproex-Natrium, Valproinsäure oder Derivate oder Salze davon; oder Terazosin, seine Salze, Hydrate oder Derivate davon.

7. Dosierform mit verzögerter Arzneimittelfreisetzung, die folgendes umfaßt:
(1) Granalien eines Arzneimittels, die mit einer Hüllzusammensetzung zur verzögerten Freisetzung, welche 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält, überzogen sind, und (2) eine Matrix mit langsamer Freisetzung, die mindestens ein Viskositätsmittel enthält, und Calciumphosphat.

8. Dosierform mit verzögerter Arzneimittelfreisetzung, die folgendes umfaßt: (1) Granalien eines Arzneimittels, die mit einer Hüllzusammensetzung zur verzögerten Freisetzung, welche 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält, überzogen sind, und (2) eine Matrix mit langsamer Freisetzung, die mindestens ein Viskositätsmittel, gewählt aus der Gruppe bestehend aus Methylcellulose, Hydroxypropylmethylcellulose, Povidon und Hydroxypropylcellulose, enthält und die desweiteren Calciumphosphat enthält.

9. Arzneimitteldosierform nach Anspruch 7 oder 8, wobei der Weichmacher aus der Gruppe bestehend aus Rizinusöl, Propylenglykol, Polyethylenglykol, Acetyltriethylcitrat, Acetyltributylcitrat, Triethylcitrat und Tributylcitrat gewählt ist, und das Trennmittel aus der Gruppe bestehend aus Magnesiumstearat, Talkum, Titandioxid und Silicagel gewählt ist, und das Viskositätsmittel aus der Gruppe bestehend aus Methylcellulose, Hydroxypropylcellulose, Povidon, und Hydroxypropylmethylcellulose gewählt ist.

10. Arzneimitteldosierform nach Anspruch 7 oder 8, die folgendes umfaßt:
2,0 bis 80 Gewichtsprozent Arzneimittelgranalien mit einem Überzug zur verzögerten Freisetzung;
bis zu 30 Gewichtsprozent Calciumphosphat;
2 bis 30 Gewichtsprozent eines ersten Viskositätsmittels und 0 bis 30 Gewichtsprozent eines zweiten Viskositätsmittels, wobei das erste und das zweite Viskositätsmittel unabhängig voneinander aus der Gruppe bestehend aus Methylcellulose, Hydroxypropylmethylcellulose, Povidon und Hydroxypropylcellulose gewählt sind.

11. Arzneimitteldosierform nach Anspruch 7 oder 8, die folgendes umfaßt:
63 bis 65 Gewichtsprozent Divalproex-Natrium-Granalien mit einem Überzug zur verzögerten Freisetzung;
3,5 bis 4,5 Gewichtsprozent Methylcellulose;
14,2 bis 14,6 Gewichtsprozent Hydroxypropylmethylcellulose;
14,2 bis 14,6 Gewichtsprozent Calciumphosphat;
0,7 bis 0,8 Gewichtsprozent Stearinsäure; und
1,5 bis 2,2 Gewichtsprozent Talkum.

12. Arzneimitteldosierform nach Anspruch 7 oder 8, die folgendes umfaßt:
2,6 bis 26,4 Gewichtsprozent Terazosinmonohydrochloriddihydratgranalien mit einem Überzug zur verzögerten Freisetzung;
etwa 2,5 Gewichtsprozent Methylcellulose;
etwa 10,5 Gewichtsprozent Hydroxypropylmethylcellulose;
etwa 34,0 Gewichtsprozent Calciumphosphat;
etwa 1,1 Gewichtsprozent Stearinsäure; und
etwa 1,9 Gewichtsprozent Talkum.

13. Verfahren zur Herstellung einer Dosierform mit verzögerter Arzneimittelfreisetzung nach Anspruch 7, das folgende Schritte umfaßt:
a) Beimischen eines Trennmittels zu einem Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
f) Mischen der überzogenen Granalien mit mindestens einem Viskositätsmittel und Calciumphosphat; und
g) Formen der Mischung, die in Schritt (f) hergestellt wurde, zu Arzneimitteldosiereinheiten.

14. Verfahren zur Herstellung einer Dosierform mit verzögerter Arzneimittelfreisetzung nach Anspruch 8, das folgende Schritte umfaßt:
a) Beimischen eines Trennmittels zu einem Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten, wobei die Zusammensetzung von Schitt (f) desweiteren Calciumphosphat enthält.

15. Verfahren nach den Ansprüchen 13 oder 14, wobei das Trennmittel in Schritt (a) Siliciumdioxid ist.

16. Verfahren nach Anspruch 13 oder 14, wobei der Weichmacher in Schritt (e) aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist; und wobei das Trennmittel in Schritt (e) aus der Gruppe bestehend aus Magnesiumstearat, Talkum, Titandioxid und Silicagel gewählt ist.

17. Verfahren nach Anspruch 13 oder 14 zur Herstellung einer Dosierform mit verzögerter Arzneimittelfreisetzung, das folgende Schritte umfaßt:
a) Beimischen von Silicagel zu einem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Vermischen der Granalien gewünschter Größe mit einer Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel enthält, das aus der Gruppe bestehend aus Magnesiumstearat, Talkum, Titandioxid und Silicagel gewählt ist, zur Herstellung überzogener Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist; und
g) Formen der Mischung, die in Schritt (f) hergestellt wurde, zu Arzneimitteldosiereinheiten.

18. Verfahren zur Herstellung einer Dosierform mit verzögerter Arzneimittelfreisetzung, das folgendes umfaßt:
a) Überziehen von Zuckerpellets mit einer Lösung des Arzneimittels zur Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel; und
f) Formen der Mischung, die nach Schritt (e) hergestellt wurde, zu Arzneimitteldosiereinheiten, wobei das Viskositätsmittel aus der Gruppe bestehend aus Methylcellulose, Hydroxypropylmethylcellulose, Povidon und Hydroxypropylcellulose gewählt ist und die Zusammensetzung aus Schritt (e) desweiteren Calciumphosphat enthält.

19. Verfahren nach Anspruch 18, wobei die Hüllzusammensetzung 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält.

20. Verfahren nach Anspruch 19, wobei der Weichmacher in Schritt (b) aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist; und wobei das Trennmittel in Schritt (b) aus der Gruppe bestehend aus Magnesiumstearat, Talkum, Titandioxid und Silicagel gewählt ist.

## Patentansprüche (Patentansprüche für folgende(n) Vertragsstaat(en): DK)

1. Dosierform mit verzögerter Arzneimittelfreisetzung, die folgendes umfaßt: Granalien eines Arzneimittels, die mit einer Hüllzusammensetzung beschichtet sind, die eine Ethylcellulose oder einen Methacryl-Methylester, einen Weichmacher und ein Trennmittel enthält, wobei die Hüllzusammensetzung Ethylcellulose, Rizinusöl als Weichmacher und Magnesiumstearat als Trennmittel enthält, und wobei das Arzneimittel Divalproex-Natrium oder Terazosinmonohydrochloriddihydrat ist.

2. Arzneimitteldosierform nach Anspruch 1, wobei der Arzneistoffpartikel mit einem Überzug zur verzögerten Freisetzung folgendes umfaßt:
62 bis 86 Gewichtsprozent Divalproex-Natrium;
2,6 bis 3,7 Gewichtsprozent Silicagel;
2,6 bis 11,6 Gewichtsprozent Ethylcellulose;
0,4 bis 1,9 Gewichtsprozent Rizinusöl;
2,6 bis 11,6 Gewichtsprozent Magnesiumstearat;
0 bis 0,09 Gewichtsprozent Farbstoff;
0,4 bis 1,0 Gewichtsprozent Povidon; und
4,5 bis 9,7 Gewichtsprozent mikrokristalline Cellulose; oder
etwa 12,9 Gewichtsprozent Terazosinmonohydrochloriddihydrat;
etwa 5,5 Gewichtsprozent Povidon;
etwa 53,6 Gewichtsprozent Zuckerpellets;
etwa 8,0 Gewichtsprozent Ethylcellulose;
etwa 1,3 Gewichtsprozent Rizinusöl;
etwa 8,0 Gewichtsprozent Magnesiumstearat; und
etwa 10,7 Gewichtsprozent mikrokristalline Cellulose.

3. Eine oral verabreichbare Arzneimitteldosiereinheit mit verzögerter Wirkstofffreisetzung, die eine Zusammensetzung von mindestens einem Viskositätsmittel mit Arzneimittelgranalien umfaßt, beschichtet mit einem Überzug zur verzögerten Arzneimittelfreisetzung, der eine Ethylcellulose oder einen Methacryl-Methylester enthält, einen Weichmacher und ein Trennmittel, wobei das Arzneimittel gewählt ist aus der Gruppe bestehend aus Divalproex-Natrium, Valproinsäure oder Derivaten oder Salzen davon; und Terazosin oder Salze oder Hydrate oder Derivate davon.

4. Verfahren zur Herstellung einer Dosierform mit verzögerter Arzneimittelfreisetzung nach Anspruch 3, das folgende Schritte umfaßt:
a) Beimischen eines Trennmittels zu einem Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten.

5. Verfahren nach Anspruch 4 zur Herstellung einer Dosierform mit verzögerter Arzneimittelfreisetzung, das folgende Schritte umfaßt:
a) Beimischen von Silicagel zu einem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Vermischen der Granalien gewünschter Größe mit einer Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel enthält, das aus der Gruppe bestehend aus Magnesiumstearat, Talkum, Titandioxid und Silicagel gewählt ist, zur Herstellung überzogener Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist; und
g) Formen der Mischung, die in Schritt (f) hergestellt wurde, zu Arzneimitteldosiereinheiten.

6. Verfahren zur Herstellung einer Dosierform mit verzögerter Arzneimittelfreisetzung nach Anspruch 3, das folgendes umfaßt:
a) Überziehen von Zuckerpellets mit einer Lösung des Arzneimittels zur Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel; und
f) Formen der Mischung, die nach Schritt (e) hergestellt wurde, zu Arzneimitteldosiereinheiten.

7. Eine oral verabreichbare Arzneimitteldosiereinheit mit verzögerter Wirkstofffreisetzung, die eine Zusammensetzung von mindestens einem Viskositätsmittel mit Arzneimittelgranalien umfaßt, beschichtet mit einem Überzug zur verzögerten Freisetzung, der eine Ethylcellulose oder einen Methacryl-Methylester enthält, einen Weichmacher und ein Trennmittel, wobei die Zusammensetzung desweiteren Calciumphosphat enthält.

8. Eine oral verabreichbare Arzneimitteldosiereinheit mit verzögerter Arzneimittelfreisetzung, die eine Zusammensetzung von mindestens einem Viskositätsmittel mit Arzneimittelgranalien umfaßt, beschichtet mit einem Überzug zur verzögerten Freisetzung, der eine Ethylcellulose oder einen Methacryl-Methylester enthält, einen Weichmacher und ein Trennmittel, wobei das Viskositätsmittel gewählt ist aus der Gruppe bestehend aus Methylcellulose, Hydroxypropylmethylcellulose, Povidon und Hydroxypropylcellulose und die Zusammensetzung desweiteren Calciumphosphat enthält.

9. Die Dosiereinheit nach Anspruch 7 oder 8, die folgendes umfaßt:
2,0 bis 80 Gewichtsprozent Arzneimittelgranalien mit einem Überzug zur verzögerten Freisetzung;
bis zu 30 Gewichtsprozent Calciumphosphat;
2 bis 30 Gewichtsprozent eines ersten Viskositätsmittels und 0 bis 30 Gewichtsprozent eines zweiten Viskositätsmittels, wobei das erste und das zweite Viskositätsmittel unabhängig voneinander aus der Gruppe bestehend aus Methylcellulose, Hydroxypropylmethylcellulose, Povidon und Hydroxypropylcellulose gewählt sind.

10. Die Dosiereinheit nach Anspruch 7 oder 8, wobei das Arzneimittel gewählt ist aus der Gruppe bestehend aus Divalproex-Natrium, Valproinsäure oder Derivate oder Salze davon; und Terazosin oder Salze oder Hydrate oder Derivate davon.

11. Die Dosiereinheit nach Anspruch 7 oder 8, die folgendes enthält:
63 bis 65 Gewichtsprozent Divalproex-Natriumgranalien mit einem Überzug zur verzögerten Freisetzung;
3,5 bis 4,5 Gewichtsprozent Methylcellulose;
14,2 bis 14,6 Gewichtsprozent Hydroxypropylmethylcellulose;
14,2 bis 14,6 Gewichtsprozent Calciumphosphat;
0,7 bis 0,8 Gewichtsprozent Stearinsäure; und
1,5 bis 2,2 Gewichtsprozent Talkum oder
2,6 bis 26,4 Gewichtsprozent Treazosinmonohydrochloriddihydatgranalien mit einem Überzug zur verzögerten Freisetzung;
etwa 2,5 Gewichtsprozent Methylcellulose;
etwa 10,5 Gewichtsprozent Hydroxypropylmethylcellulose;
etwa 34,0 Gewichtsprozent Calciumphosphat;
etwa 1,1 Gewichtsprozent Stearinsäure; und
etwa 1,9 Gewichtsprozent Talkum.

12. Verfahren zur Herstellung einer Dosierform mit verzögerter Arzneimittelfreisetzung nach Anspruch 7 oder 8, das folgende Schritte umfaßt:
a) Beimischen eines Trennmittels zu einem Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten, wobei das Viskositätsmittel gewählt ist aus der Gruppe bestehend aus Methylcellulose, Hydroxypropylmethylcellulose, Povidon und Hydroxypropylcellulose und die Zusammensetzung aus Schritt (f) desweiteren Calciumphosphat enthält.

13. Verfahren nach Anspruch 12, wobei das Trennmittel in Schritt (a) Siliciumdioxid ist.

14. Verfahren nach Anspruch 12, wobei die Hüllzusammensetzung 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält.

15. Verfahren nach Anspruch 12, wobei der Weichmacher in Schritt (e) aus der Gruppe bestehend aus Rizinusöl, Propylenglykol, Polyethylenglykol, Acetyltriethylcitrat, Acetyltributylcitrat, Triethylcitrat und Tributylcitrat gewählt ist; und wobei das Trennmittel in Schritt (e) aus der Gruppe bestehend aus Magnesiumstearat, Talkum, Titandioxid und Silicagel gewählt ist.

16. Verfahren nach Anspruch 12 zur Herstellung einer Dosierform mit verzögerter Arzneimittelfreisetzung, das folgende Schritte umfaßt:
a) Beimischen von Silicagel zu diesem Arzneimittel;
b) Zusetzen eines Lösungsmittels zu dieser Beimischung unter ständigem Mischen, zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel enthält, das aus der Gruppe bestehend aus Magnesiumstearat, Talkum, Titandioxid und Silicagel gewählt ist, zur Herstellung von überzogenen Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist, und mit Calciumphosphat; und
g) Formen der Mischung, die in Schritt (f) hergestellt wurde, zu Arzneimitteldosiereinheiten.

17. Verfahren nach Anspruch 12 oder 16, wobei das Arzneimittel gewählt ist aus der Gruppe bestehend aus Divalproex-Natrium, Valproinäure oder Derivate oder Salze davon; oder Terazosin, seine Salze, Hydrate oder Derivate davon.

18. Verfahren nach Anspruch 7 oder 8 zur Herstellung einer Dosierform mit verzögerter Arzneimittelfreisetzung, das folgendes umfaßt:
a) Überziehen von Zuckerpellets mit einer Lösung des Arzneimittels unter Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel und mit Calciumphosphat; und
f) Formen der Mischung, die nach Schritt (e) hergestellt wurde, zu Arzneimitteldosiereinheiten.

19. Verfahren nach Anspruch 18, wobei die Hüllzusammensetzung 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält.

20. Verfahren nach Anspruch 18, wobei der Weichmacher in Schritt (b) aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist; und wobei das Trennmittel in Schritt (b) aus der Gruppe bestehend aus Magnesiumstearat, Talkum, Titandioxid und Silicagel gewählt ist.

21. Verfahren nach Anspruch 18, wobei das Arzneimittel gewählt ist aus der Gruppe bestehend aus Divalproex-Natrium, Valproinsäure oder Derivate oder Salze davon; oder Terazosin, seine Salze, Hydrate oder Derivate davon.

## Patentansprüche (Patentansprüche für folgende(n) Vertragsstaat(en): ES, GR)

1. Verfahren zur Herstellung einer Dosierform mit verzögerter Arzneimittelfreisetzung, das folgendes umfaßt:
(1) Granalien eines Arzneimittels, die mit einer Hüllzusammensetzung zur verzögerten Freisetzung überzogen sind, die 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält, und (2) eine Matrix mit langsamer Freisetzung, die mindestens ein Viskositätsmittel enthält, wobei das Verfahren folgendes umfaßt:
a) Beimischen eines Trennmittels zu einem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten, wobei das Arzneimittel gewählt ist aus der Gruppe bestehend aus Divalproex-Natrium, Valproinsäure oder Derivaten oder Salzen davon; oder Terazosin, seine Salze, Hydrate oder Derivate davon.

2. Verfahren nach Anspruch 1 zur Herstellung einer Dosierform mit verzögerter Arzneimittelfreisetzung, das folgende Schritte umfaßt:
a) Beimischen von Silicagel zu diesem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Vermischen der Granalien gewünschter Größe mit einer Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel umfaßt, das aus der Gruppe bestehend aus Magnesiumstearat, Talkum, Titandioxid und Silicagel gewählt ist, zur Herstellung überzogener Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist; und
g) Formen der Mischung, die in Schritt (f) hergestellt wurde, zu Arzneimitteldosiereinheiten.

3. Verfahren nach Anspruch 1 zur Herstellung einer Arzneiform, wobei der Arzneistoffpartikel mit einem Überzug zur verzögerten Freisetzung folgendes enthält:
von 62 bis 86 Gewichtsprozent Divalproex-Natrium;
von 2,6 bis 3,7 Gewichtsprozent Silicagel;
von 2,6 bis 11,6 Gewichtsprozent Ethylcellulose;
von 0,4 bis 1,9 Gewichtsprozent Rizinusöl;
von 2,6 bis 11,6 Gewichtsprozent Magnesiumsteärat;
von 0 bis 0,09 Gewichtsprozent Farbstoff;
von 0,4 bis 1,0 Gewichtsprozent Povidon; und
von 4,5 bis 9,7 Gewichtsprozent mikrokristalline Cellulose.

4. Verfahren nach Anspruch 1 zur Herstellung einer Arzneiform, wobei der Arzneistoffpartikel mit einem Überzug zur verzögerten Freisetzung folgendes enthält:
etwa 12,9 Gewichtsprozent Terazosinmonohydrochloriddihydrat;
etwa 5,5 Gewichtsprozent Povidon;
etwa 53,6 Gewichtsprozent Zuckerpellets;
etwa 8,0 Gewichtsprozent Ethylcellulose;
etwa 1,3 Gewichtsprozent Rizinusöl;
etwa 8,0 Gewichtsprozent Magnesiumstearat; und
etwa 10,7 Gewichtsprozent mikrokristalline Cellulose.

5. Verfahren zur Herstellung einer Dosierform mit verzögerter Arzneimittelfreisetzung, das folgendes umfaßt:
a) Überziehen von Zuckerpellets mit einer Lösung des Arzneimittels unter Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel; und
f) Formen der Mischung, die nach Schritt (e) hergestellt wurde, zu Arzneimitteldosiereinheiten, wobei das Arzneimittel gewählt ist aus der Gruppe bestehend aus Divalproex-Natrium, Valproinsäure oder Derivaten oder Salzen davon; oder Terazosin, seine Salze, Hydrate oder Derivate davon.

6. Verfahren zur Herstellung einer Dosierform mit verzögerter Arzneimittelfreisetzung, das folgendes umfaßt:
(1) Granalien eines Arzneimittels, die mit einer Hüllzusammensetzung zur verzögerten Freisetzung überzogen sind, die 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält, und (2) eine Matrix mit langsamer Freisetzung, die mindestens ein Viskositätsmittel enthält, wobei das Verfahren folgendes umfaßt:
a) Beimischen eines Trennmittels zu einem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Mischen der Granalien gewünschter Größe mit einer Hüllzusammensetzung, zur Herstellung überzogener Granalien;
f) Mischen der überzogenen Granalien mit mindestens einem Viskositätsmittel; und
g) Formen der Mischung, die in Schritt (f) hergestellt wurde, zu Arzneimitteldosiereinheiten, wobei das Viskositätsmittel gewählt ist aus der Gruppe bestehend aus Methylcellulose, Hydroxypropylmethylcellulose, Povidon und Hydroxypropylcellulose und die Zusammensetzung aus Schritt (f) desweiteren Calciumphosphat enthält.

7. Verfahren nach Anspruch 6, wobei das Trennmittel in Schritt (a) Siliciumdioxid ist.

8. Verfahren nach Anspruch 6, wobei der Weichmacher in Schritt (e) aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist; und wobei das Trennmittel in Schritt (e) aus der Gruppe bestehend aus Magnesiumstearat, Talkum, Titandioxid und Silicagel gewählt ist.

9. Verfahren nach Anspruch 6 zur Herstellung einer Dosierform mit verzögerter Arzneimittelfreisetzung, das folgende Schritte umfaßt:
a) Beimischen von Silicagel zu einem Arzneimittel;
b) Zusetzen eines Lösungsmittels zu dieser Beimischung unter ständigem Mischen zur Herstellung von Arzneimittelgranalien;
c) Trocknen der Arzneimittelgranalien;
d) Sieben und Klassieren der Granalien, um Granalien gewünschter Größe zu erhalten;
e) Vermischen der Granalien gewünschter Größe mit der Hüllzusammensetzung, wobei die Hüllzusammensetzung eine Ethylcellulose oder einen Methacryl-Methylester, einen Weichmacher, der aus der Gruppe bestehend aus Rizinusöl, Propylenglykol, Polyethylenglykol, Acetyltriethylcitrat, Acetyltributylcitrat, Trietyhlcitrat und Tributylcitrat gewählt ist, und ein Trennmittel enthält, das aus der Gruppe bestehend aus Magnesiumstearat, Talkum, Titandioxid und Silicagel gewählt ist, zur Herstellung überzogener Granalien;
f) Mischen der überzogenen Granalien mit mindestens einem Viskositätsmittel, das aus der Gruppe bestehend aus Methylcellulose, Hydroxypropylmethylcellulose, Povidon und Hydroxypropylcellulose gewählt ist; und
g) Formen der Mischung, die in Schritt (f) hergestellt wurde, zu Arzneimitteldosiereinheiten.

10. Verfahren nach Anspruch 6 zur Herstellung einer Arzneiform, das folgendes umfaßt: 2,0 bis 80 Gewichtsprozent Arzneimittelgranalien mit verzögerter Arzneimittelfreisetzung; bis zu 30 Gewichtsprozent Calciumphosphat; 2 bis 30 Gewichtsprozent eines ersten Viskositätsmittels und 0 bis 30 Gewichtsprozent eines zweiten Viskositätsmittels, wobei das erste und zweite Viskositätsmittel unabhängig voneinander gewählt sind aus der Gruppe bestehend aus Methylcellulose, Hydroxpropylmethylcellulose, Povidon und Hydroxypropylcellulose.

11. Verfahren nach Anspruch 5 zur Herstellung einer Arzneiform, das folgendes umfaßt:
63 bis 65 Gewichtsprozent Divalproex-Natriumgranalien mit einem Überzug zur verzögerten Freisetzung;
3,5 bis 4,5 Gewichtsprozent Methylcellulose;
14,2 bis 14,6 Gewichtsprozent Hydroxypropylmethylcellulose;
14,2 bis 14,6 Gewichtsprozent Calciumphosphat;
0,7 bis 0,8 Gewichtsprozent Stearinsäure; und
1,5 bis 2,2 Gewichtsprozent Talkum.

12. Verfahren nach Anspruch 5 zur Herstellung einer Arzneiform, das folgendes umfaßt:
2,6 bis 26,4 Gewichtsprozent Terazosinmonohydrochloriddihydratgranalien mit einem Überzug zur verzögerten Freisetzung;
etwa 2,5 Gewichtsprozent Methylcellulose;
etwa 10,5 Gewichtsprozent Hydroxypropylmethylcellulose;
etwa 34,0 Gewichtsprozent Calciumphosphat;
etwa 1,1 Gewichtsprozent Stearinsäure; und
etwa 1,9 Gewichtsprozent Talkum.

13. Verfahren zur Herstellung einer Dosierform mit verzögerter Arzneimittelfreisetzung, das folgendes umfaßt:
a) Überziehen von Zuckerpellets mit einer Lösung des Arzneimittels zur Bildung von Arzneimittelgranalien;
b) Mischen der Granalien mit einer Hüllzusammensetzung, wobei die Hüllzusammensetzung eine Ethylcellulose oder einen Methacryl-Methylester, einen Weichmacher und ein Trennmittel enthält, zur Herstellung überzogener Granalien;
c) Trocknen der überzogenen Granalien;
d) Sieben und Klassieren der überzogenen Granalien, um Granalien gewünschter Größe zu erhalten;
e) Mischen der Granalien gewünschter Größe mit mindestens einem Viskositätsmittel; und
f) Formen der Mischung, die nach Schritt (e) hergestellt wurde, zu Arzneimitteldosiereinheiten, wobei das Viskositätsmittel aus der Gruppe bestehend aus Methylcellulose, Hydroxypropylmethylcellulose, Povidon und Hydroxypropylcellulose gewählt ist und die Zusammensetzung aus Schritt (e) desweiteren Calciumphosphat enthält.

14. Verfahren nach Anspruch 13, wobei die Hüllzusammensetzung 2 bis 20 Gewichtsprozent (g/ml x 100) einer Ethylcellulose oder eines Methacryl-Methylesters, 0,1 bis 5,0 Gewichtsprozent (g/ml x 100) eines Weichmachers und 0,5 bis 20 Gewichtsprozent (g/ml x 100) eines Trennmittels enthält.

15. Verfahren nach Anspruch 14, wobei der Weichmacher in Schritt (b) aus der Gruppe bestehend aus Rizinusöl, Propylenglykol, Polyethylenglykol, Acetyltriethylzitrat, Acetyltributylzitrat, Triethylzitrat und Tributylzitrat gewählt ist; und wobei das Trennmittel aus Schritt (b) aus der Gruppe bestehend aus Magnesiumstearat, Talkum, Titandioxid und Silicagel gewählt ist.

16. Verfahren nach Anspruch 13, wobei das Arzneimittel gewählt ist aus der Gruppe bestehend aus Divalproex-Natrium, Valproinsäure oder Derivaten oder Salzen davon; oder Terazosin, seine Salze, Hydrate oder Derivate davon.

## Revendications (Revendications pour l'(les) Etat(s) contractant(s) suivant(s): AT, BE, CH, DE, FR, GB, IT, LI, LU, NL, SE)

1. Forme pharmaceutique d'un médicament à libération prolongée comprenant (1) des granules d'un médicament revêtus d'une composition de revêtement à libération prolongée comprenant de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif et (2) une matrice à libération lente comprenant au moins un agent de viscosité, dans laquelle le médicament est choisi parmi le divalproex de sodium, l'acide valproïque ou ses amides ou esters ou sels, ou la térazosine ou ses sels, hydrates ou dérivés.

2. Forme pharmaceutique d'un médicament selon la revendication 1, dans laquelle la particule de médicament revêtue à libération prolongée comprend:
de 62 à 86 % en poids de divalproex de sodium;
de 2,6 à 3,7 % en poids de gel de silice;
de 2,6 à 11,6 % en poids d'éthylcellulose;
de 0,4 à 1,9 % en poids d'huile de ricin;
de 2,6 à 11,6 % en poids de stéarate de magnésium;
de 0 à 0,09 % en poids d'un colorant;
de 0,4 à 1,0 % en poids de povidone ; et
de 4,5 à 9,7 % en poids de cellulose microcristalline.

3. Forme pharmaceutique d'un médicament selon la revendication 1, dans laquelle la particule de médicament revêtue à libération prolongée comprend:
environ 12,9 % en poids de monochlorhydrate de térazosine dihydraté;
environ 5,5 % en poids de povidone;
environ 53,6 % en poids de sphères de sucre ;
environ 8,0 % en poids d'éthylcellulose;
environ 1,3 % en poids d'huile de ricin;
environ 8,0 % en poids de stéarate de magnésium; et
environ 10,7 % en poids de cellulose microcristalline.

4. Procédé pour la préparation d'une forme pharmaceutique d'un médicament à libération prolongée selon la revendication 1, comprenant les étapes suivantes:
a) on mélange ledit médicament avec un agent antiadhésif;
b) on ajoute un solvant audit mélange en agitant constamment pour produire des granules de médicament ;
c) on sèche lesdits granules de médicament;
d) on tamise et on classe lesdits granules pour obtenir des granules de taille désirée ;
e) on mélange lesdits granules de taille désirée avec une composition de revêtement, ladite composition de revêtement comprenant une éthylcellulose ou un ester méthacrylique de méthyle, un plastifiant et un agent antiadhésif, pour produire des granules revêtus ;
f) on mélange lesdits granules revêtus avec au moins un agent de viscosité ; et
g) on met ledit mélange produit dans l'étape (f) sous forme d'unités de prise du médicament.

5. Procédé selon la revendication 4, pour la préparation d'une forme pharmaceutique d'un médicament, comprenant les étapes suivantes:
a) on mélange ledit médicament avec du gel de silice ;
b) on ajoute un solvant audit mélange en agitant constamment pour produire des granules de médicament;
c) on sèche lesdits granules de médicament;
d) on tamise et on classe lesdits granules pour obtenir des granules de taille désirée ;
e) on mélange lesdits granules de taille désirée avec une composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice, pour produire des granules revêtus
f) on mélange lesdits granules revêtus avec au moins un agent de viscosité choisi parmi la méthylcellulose, l'hydroxypropylméthylcellulose, la povidone et l'hydroxypropylcellulose ; et
g) on met ledit mélange produit dans l'étape (f) sous la forme d'unités de prise du médicament.

6. Procédé pour la préparation d'une forme pharmaceutique d'un médicament à libération prolongée, comprenant les étapes suivantes:
a) on enduit des sphères de sucre d'une solution du médicament, pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus
d) on tamise et on classe lesdits granules revêtus pour obtenir des granules revêtus de taille désirée;
e) on mélange lesdits granules revêtus de taille désirée avec au moins un agent de viscosité ; et
f) on met ledit mélange produit dans l'étape (e) sous la forme d'unités de prise du médicament,
dans lequel ledit médicament est choisi parmi le divalproex de sodium, l'acide valproïque ou ses dérivés ou sels, ou la térazosine, ses sels, hydrates ou dérivés.

7. Forme pharmaceutique d'un médicament à libération prolongée comprenant (1) des granules d'un médicament revêtus d'une composition de revêtement à libération prolongée comprenant de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif et (2) une matrice à libération lente comprenant au moins un agent de viscosité et du phosphate de calcium.

8. Forme pharmaceutique d'un médicament à libération prolongée comprenant (1) des granules d'un médicament revêtus d'une composition de revêtement à libération prolongée comprenant de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif et (2) une matrice à libération lente comprenant au moins un agent de viscosité choisi parmi la méthylcellulose, l'hydroxypropylméthylcellulose, la povidone et l'hydroxypropylcellulose et comprenant en outre du phosphate de calcium.

9. Forme pharmaceutique d'un médicament selon la revendication 7 ou 8, dans laquelle ledit plastifiant est choisi parmi l'huile de ricin, le propylèneglycol, le polyéthylèneglycol, l'acétylcitrate de triéthyle, l'acétylcitrate de tributyle, le citrate de triéthyle et le citrate de tributyle et ledit agent antiadhésif est choisi parmi le stéarate de magnésium, le talc, le dioxyde de titane et le gel de silice et ledit agent de viscosité est choisi parmi la méthylcellulose, l'hydroxypropylcellulose, la povidone et l'hydroxypropylméthylcellulose.

10. Forme pharmaceutique d'un médicament selon la revendication 7 ou 8, comprenant
de 2,0 à 80 % en poids de granules de médicament revêtus à libération prolongée;
jusqu'à 30 % en poids de phosphate de calcium;
de 2 à 30 % en poids d'un premier agent de viscosité et de 0 à 30 % en poids d'un second agent de viscosité,
dans laquelle lesdits premier et second agents de viscosité sont choisis indépendamment parmi la méthylcellulose, l'hydroxypropylméthylcellulose, la povidone et l'hydroxypropylcellulose.

11. Forme pharmaceutique d'un médicament selon la revendication 7 ou 8, comprenant:
de 63 à 65 % en poids de granules de divalproex de sodium revêtus à libération prolongée;
de 3,5 à 4,5 % en poids de méthylcellulose;
de 14,2 à 14,6 % en poids d'hydroxypropylméthylcellulose;
de 14,2 à 14,6 % en poids de phosphate de calcium;
de 0,7 à 0,8 % en poids d'acide stéarique ; et
de 1,5 à 2,2 % en poids de talc.

12. Forme pharmaceutique d'un médicament selon la revendication 7 ou 8, comprenant:
de 2,6 à 26,4 % en poids de granules de monochlorhydrate de térazosine dihydraté revêtus à libération prolongée;
environ 2,5 % en poids de méthylcellulose;
environ 10,5 % en poids d'hydroxypropylméthylcellulose;
environ 34,0 % en poids de phosphate de calcium;
environ 1,1 % en poids d'acide stéarique ; et
environ 1,9 % en poids de talc.

13. Procédé pour la préparation d'une forme pharmaceutique d'un médicament à libération prolongée selon la revendication 7, comprenant les étapes suivantes:
a) on mélange un médicament avec un agent antiadhésif;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant et un agent antiadhésif, pour produire des granules revêtus;
f) on mélange lesdits granules revêtus avec au moins un agent de viscosité et du phosphate de calcium ; et
g) on met ledit mélange produit dans l'étape (f) sous forme d'unités de prise du médicament.

14. Procédé pour la préparation d'une forme pharmaceutique d'un médicament à libération prolongée selon la revendication 8, comprenant les étapes suivantes :
a) on mélange un médicament avec un agent antiadhésif;
b) on ajoute un solvant audit mélange en agitant constamment pour produire des granules de médicament ;
c) on sèche lesdits granules de médicament;
d) on tamise et on classe lesdits granules pour obtenir des granules de taille désirée ;
e) on mélange lesdits granules de taille désirée avec une composition de revêtement, ladite composition de revêtement comprenant une éthylcellulose ou un ester méthacrylique de méthyle, un plastifiant et un agent antiadhésif, pour produire des granules revêtus;
f) on mélange lesdits granules revêtus avec au moins un agent de viscosité ; et
g) on met ledit mélange produit dans l'étape (f) sous forme d'unités de prise du médicament,
dans lequel la composition de l'étape (f) comprend en outre du phosphate de calcium.

15. Procédé selon la revendication 13 ou 14, dans lequel ledit agent anti-adhésif dans l'étape (a) est la silice.

16. Procédé selon la revendication 13 ou 14, dans lequel ledit plastifiant dans l'étape (e) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (e) est choisi parmi le stéarate de magnésium, le talc, le dioxyde de titane et le gel de silice.

17. Procédé selon la revendication 13 ou 14, pour la préparation d'une forme pharmaceutique d'un médicament, comprenant les étapes suivantes:
a) on mélange un médicament avec du gel de silice;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice, pour produire des granules revêtus ;
f) on mélange lesdits granules revêtus avec au moins un agent de viscosité choisi parmi la méthylcellulose, l'hydroxypropylméthylcellulose, la povidone et l'hydroxypropylcellulose ; et
g) on met ledit mélange produit dans l'étape (f) sous la forme d'unités de prise du médicament.

18. Procédé pour la préparation d'une forme pharmaceutique d'un médicament à libération prolongée, comprenant les étapes suivantes:
a) on enduit des sphères de sucre d'une solution du médicament, pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus;
d) on tamise et on classe lesdits granules revêtus pour obtenir des granules revêtus de taille désirée;
e) on mélange lesdits granules revêtus de taille désirée avec au moins un agent de viscosité ; et
f) on met ledit mélange produit dans l'étape (e) sous la forme d'unités de prise du médicament,
dans lequel ledit agent de viscosité est choisi parmi la méthylcellulose, l'hydroxypropylméthylcellulose, la povidone et l'hydroxypropylcellulose et la composition de l'étape (e) comprend en outre du phosphate de calcium.

19. Procédé selon la revendication 18, dans lequel ladite composition de revêtement comprend de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif.

20. Procédé selon la revendication 19, dans lequel ledit plastifiant de l'étape (b) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (b) est choisi parmi le stéarate de magnésium, le talc, le dioxyde de titane et le gel de silice.

## Revendications (Revendications pour l'(les) Etat(s) contractant(s) suivant(s): DK)

1. Médicament revêtu à libération prolongée comprenant des granules dudit médicament revêtus par une composition de revêtement comprenant une éthylcellulose ou un ester méthacrylique de méthyle, un plastifiant et un agent antiadhésif, dans lequel la composition de revêtement comprend de l'éthylcellulose, de l'huile de ricin comme dit plastifiant et du stéarate de magnésium comme dit agent antiadhésif et dans lequel ledit médicament est le divalproex de sodium ou le monochlorhydrate de térazosine dihydraté.

2. Particule de médicament revêtue à libération prolongée selon la revendication 1, comprenant:
de 62 à 86 % en poids de divalproex de sodium;
de 2,6 à 3,7 % en poids de gel de silice;
de 2,6 à 11,6 % en poids d'éthylcellulose;
de 0,4 à 1,9 % en poids d'huile de ricin;
de 2,6 à 11,6 % en poids de stéarate de magnésium;
de 0 à 0,09 % en poids d'un colorant;
de 0,4 à 1,0 % en poids de povidone ; et
de 4,5 à 9,7 % en poids de cellulose microcristalline ; ou bien
environ 12,9 % en poids de monochlorhydrate de térazosine dihydraté;
environ 5,5 % en poids de povidone;
environ 53,6% en poids de sphères de sucre;
environ 8,0 % en poids d'éthylcellulose;
environ 1,3 % en poids d'huile de ricin;
environ 8,0 % en poids de stéarate de magnésium ; et
environ 10,7 % en poids de cellulose microcristalline.

3. Unité de prise d'un médicament à libération prolongée pour l'administration orale, comprenant une composition d'au moins un agent de viscosité avec des granules de médicament revêtus par un revêtement à libération prolongée comprenant une éthylcellulose ou un ester méthacrylique de méthyle, un plastifiant et un agent antiadhésif, dans laquelle ledit médicament est choisi parmi le divalproex de sodium, l'acide valproïque ou ses dérivés ou ses sels ; et la térazosine ou ses sels ou hydrates ou dérivés.

4. Procédé pour la préparation d'une forme pharmaceutique d'un médicament à libération prolongée selon la revendication 3, comprenant les étapes suivantes:
a) on mélange un médicament avec un agent antiadhésif;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant et un agent antiadhésif, pour produire des granules revêtus;
f) on mélange lesdits granules revêtus avec au moins un agent de viscosité ; et
g) on met ledit mélange produit dans l'étape (f) sous forme d'unités de prise du médicament.

5. Procédé selon la revendication 4 pour la préparation d'une forme pharmaceutique d'un médicament à libération prolongée, comprenant les étapes suivantes:
a) on mélange ledit médicament avec du gel de silice;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice, pour produire des granules revêtus;
f) on mélange lesdits granules revêtus avec au moins un agent de viscosité choisi parmi la méthylcellulose, l'hydroxypropylméthylcellulose, la povidone et l'hydroxypropylcellulose ; et
g) on met ledit mélange produit dans l'étape (f) sous la forme d'unités de prise du médicament.

6. Procédé pour la préparation d'une forme pharmaceutique d'un médicament à libération prolongée selon la revendication 3, comprenant les étapes suivantes:
a) on enduit des sphères de sucre d'une solution du médicament pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus;
d) on tamise et on classe lesdits granules revêtus pour obtenir des granules revêtus de taille désirée;
e) on mélange lesdits granules revêtus de taille désirée avec au moins un agent de viscosité ; et
f) on met ledit mélange produit dans l'étape (e) sous la forme d'unités de prise du médicament.

7. Unité de prise d'un médicament à libération prolongée pour l'administration orale, comprenant une composition d'au moins un agent de viscosité avec des granules de médicament revêtus par un revêtement à libération prolongée comprenant une éthylcellulose ou un ester méthacrylique de méthyle, un plastifiant et un agent antiadhésif, dans laquelle la composition comprend en outre du phosphate de calcium.

8. Unité de prise d'un médicament à libération prolongée pour l'administration orale comprenant une composition d'au moins un agent de viscosité avec des granules de médicament revêtus par un revêtement à libération prolongée comprenant une éthylcellulose ou un ester méthacrylique de méthyle, un plastifiant et un agent antiadhésif, dans laquelle ledit agent de viscosité est choisi parmi la méthylcellulose, l'hydroxypropylméthylcellulose, la povidone et l'hydroxypropylcellulose et comprenant en outre du phosphate de calcium dans ladite composition.

9. Unité de prise selon la revendication 7 ou 8, comprenant:
de 2,0 à 80 % en poids de granules de médicaments revêtus à libération prolongée;
jusqu'à 30 % en poids de phosphate de calcium;
de 2 à 30 % en poids d'un premier agent de viscosité et de 0 à 30 % en poids d'un second agent de viscosité,
dans laquelle lesdits premier et second agents de viscosité sont choisis indépendamment parmi la méthylcellulose, l'hydroxypropylméthylcellulose, la povidone et l'hydroxypropylcellulose.

10. Unité de prise selon la revendication 7 ou 8, dans laquelle ledit médicament est choisi parmi le divalproex de sodium, l'acide valproïque ou ses dérivés ou ses sels ; et la térazosine ou ses sels ou hydrates ou dérivés.

11. Unité de prise selon la revendication 7 ou 8, comprenant:
de 63 à 65 % en poids de granules de divalproex de sodium revêtus à libération prolongée;
de 3,5 à 4,5 % en poids de méthylcellulose;
de 14,2 à 14,6 % en poids d'hydroxypropylméthylcellulose;
de 14,2 à 14,6 % en poids de phosphate de calcium;
de 0,7 à 0,8 % en poids d'acide stéarique ; et
de 1,5 à 2,2 % en poids de talc ; ou bien
de 2,6 à 26,4 % en poids de granules de monochlorhydrate de térazosine dihydraté revêtus à libération prolongée;
environ 2,5 % en poids de méthylcellulose
environ 10,5 % en poids d'hydroxypropylméthylcellulose;
environ 34,0 % en poids de phosphate de calcium;
environ 1,1 % en poids d'acide stéarique ; et
environ 1,9 % en poids de talc.

12. Procédé pour la préparation d'une forme pharmaceutique d'un médicament à libération prolongée selon la revendication 7 ou 8, comprenant les étapes suivantes:
a) on mélange un médicament avec un agent antiadhésif;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant et un agent antiadhésif, pour produire des granules revêtus;
f) on mélange lesdits granules revêtus avec au moins un agent de viscosité ; et
g) on met ledit mélange produit dans l'étape (f) sous forme d'unités de prise du médicament,
dans lequel ledit agent de viscosité est choisi parmi la méthylcellulose, l'hydroxypropylméthylcellulose, la povidone et l'hydroxypropylcellulose et la composition de l'étape (f) comprend en outre du phosphate de calcium.

13. Procédé selon la revendication 12, dans lequel ledit agent anti-adhésif dans l'étape (a) est la silice.

14. Procédé selon la revendication 12, dans lequel ladite composition de revêtement comprend de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif.

15. Procédé selon la revendication 12, dans lequel ledit plastifiant de l'étape (e) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (e) est choisi parmi le stéarate de magnésium, le talc, le dioxyde de titane et le gel de silice.

16. Procédé selon la revendication 12 pour la préparation d'une forme pharmaceutique d'un médicament à libération prolongée, comprenant les étapes suivantes:
a) on mélange ledit médicament avec du gel de silice;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice, pour produire des granules revêtus;
f) on mélange lesdits granules revêtus avec au moins un agent de viscosité choisi parmi la méthylcellulose, l'hydroxypropylméthylcellulose, la povidone et l'hydroxypropylcellulose et avec du phosphate de calcium ; et
g) on met ledit mélange produit dans l'étape (f) sous la forme d'unités de prise du médicament.

17. Procédé selon la revendication 12 ou 16, dans lequel ledit médicament est choisi parmi le divalproex de sodium, l'acide valproïque ou ses dérivés ou ses sels ; ou la térazosine, ses sels, hydrates ou dérivés.

18. Procédé pour la préparation d'une forme pharmaceutique d'un médicament à libération prolongée selon la revendication 7 ou 8, comprenant les étapes suivantes:
a) on enduit des sphères de sucre d'une solution du médicament pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus;
d) on tamise et on classe lesdits granules revêtus pour obtenir des granules revêtus de taille désirée;
e) on mélange lesdits granules revêtus de taille désirée avec au moins un agent de viscosité et du phosphate de calcium ; et
f) on met ledit mélange produit dans l'étape (e) sous la forme d'unités de prise du médicament.

19. Procédé selon la revendication 18, dans lequel ladite composition de revêtement comprend de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif.

20. Procédé selon la revendication 18, dans lequel ledit plastifiant de l'étape (b) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (b) est choisi parmi le stéarate de magnésium, le talc, le dioxyde de titane et le gel de silice.

21. Procédé selon la revendication 18, dans lequel ledit médicament est choisi parmi le divalproex de sodium, l'acide valproïque ou ses dérivés ou ses sels; ou la térazosine, ses sels, hydrates ou dérivés.

## Revendications (Revendications pour l'(les) Etat(s) contractant(s) suivant(s): ES , GR)

1. Procédé pour la préparation d'une forme pharmaceutique d'un médicament à libération prolongée comprenant:
(1) des granules d'un médicament revêtus d'une composition de revêtement à libération prolongée comprenant de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif et (2) une matrice à libération lente comprenant au moins un agent de viscosité, ledit procédé comprenant les étapes suivantes:
a) on mélange un médicament avec un agent antiadhésif;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une dite composition de revêtement pour produire des granules revêtus;
f) on mélange lesdits granules revêtus avec au moins un agent de viscosité ; et
g) on met ledit mélange produit dans l'étape (f) sous forme d'unités de prise du médicament,
dans lequel ledit médicament est choisi parmi le divalproex de sodium, l'acide valproïque ou ses dérivés ou ses sels ; ou la térazosine, ses sels, hydrates ou dérivés.

2. Procédé selon la revendication 1 pour la préparation d'une forme pharmaceutique d'un médicament à libération prolongée, comprenant les étapes suivantes:
a) on mélange ledit médicament avec du gel de silice;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice pour produire des granules revêtus;
f) on mélange lesdits granules revêtus avec au moins un agent de viscosité choisi parmi la méthylcellulose, l'hydroxypropylméthylcellulose, la povidone et l'hydroxypropylcellulose ; et
g) on met ledit mélange produit dans l'étape (f) sous la forme d'unités de prise du médicament.

3. Procédé selon la revendication 1 pour la préparation d'une forme pharmaceutique d'un médicament, dans lequel la particule d'un médicament revêtue à libération prolongée comprend:
de 62 à 86 % en poids de divalproex de sodium;
de 2,6 à 3,7 % en poids de gel de silice;
de 2,6 à 11,6 % en poids d'éthylcellulose;
de 0,4 à 1,9 % en poids d'huile de ricin;
de 2,6 à 11,6 % en poids de stéarate de magnésium;
de 0 à 0,09 % en poids d'un colorant;
de 0,4 à 1,0 % en poids de povidone ; et
de 4,5 à 9,7 % en poids de cellulose microcristalline.

4. Procédé selon la revendication 1 pour la préparation d'une forme pharmaceutique d'un médicament, dans lequel la particule d'un médicament revêtue à libération prolongée comprend:
environ 12,9 % en poids de monochlorhydrate de térazosine dihydraté;
environ 5,5 % en poids de povidone;
environ 53,6 % en poids de sphères de sucre;
environ 8,0 % en poids d'éthylcellulose;
environ 1,3 % en poids d'huile de ricin;
environ 8,0 % en poids de stéarate de magnésium ; et
environ 10,7 % en poids de cellulose microcristalline.

5. Procédé pour la préparation d'une forme pharmaceutique d'un médicament à libération prolongée, comprenant les étapes suivantes:
a) on enduit des sphères de sucre d'une solution du médicament pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus;
d) on tamise et on classe lesdits granules revêtus pour obtenir des granules revêtus de taille désirée;
e) on mélange lesdits granules revêtus de taille désirée avec au moins un agent de viscosité ; et
f) on met ledit mélange produit dans l'étape (e) sous la forme d'unités de prise du médicament,
dans lequel ledit médicament est choisi parmi le divalproex de sodium, l'acide valproïque ou ses dérivés ou ses sels ; ou la térazosine, ses sels, hydrates ou dérivés.

6. Procédé pour la préparation d'une forme pharmaceutique d'un médicament à libération prolongée comprenant:
(1) des granules d'un médicament revêtus d'une composition de revêtement à libération prolongée comprenant de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif et (2) une matrice à libération lente comprenant au moins un agent de viscosité, ledit procédé comprenant les étapes suivantes:
a) on mélange un médicament avec un agent antiadhésif;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une dite composition de revêtement pour produire des granules revêtus;
f) on mélange lesdits granules revêtus avec au moins un agent de viscosité ; et
g) on met ledit mélange produit dans l'étape (f) sous forme d'unités de prise du médicament,
dans lequel ledit agent de viscosité est choisi parmi la méthylcellulose, l'hydroxypropylméthylcellulose, la povidone et l'hydroxypropylcellulose et la composition de l'étape (f) comprend en outre du phosphate de calcium.

7. Procédé selon la revendication 6, dans lequel ledit agent antiadhésif dans l'étape (a) est la silice.

8. Procédé selon la revendication 6, dans lequel ledit plastifiant dans l'étape (e) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (e) est choisi parmi le stéarate de magnésium, le talc, le dioxyde de titane et le gel de silice.

9. Procédé selon la revendication 6 pour la préparation d'une forme pharmaceutique d'un médicament à libération retardée, comprenant les étapes suivantes:
a) on mélange un médicament avec du gel de silice;
b) on ajoute un solvant audit mélange en agitant constamment pour produire des granules de médicament;
c) on sèche lesdits granules de médicament;
d) on tamise et on classe lesdits granules pour obtenir des granules de taille désirée;
e) on mélange lesdits granules de taille désirée avec une dite composition de revêtement, ladite composition de revêtement comprenant une éthylcellulose ou un ester méthacrylique de méthyle, un plastifiant choisi parmi l'huile de ricin, le propylèneglycol, le polyéthylèneglycol, l'acétylcitrate de triéthyle, l'acétylcitrate de tributyle, le citrate de triéthyle et le citrate de tributyle et un agent antiadhésif choisi parmi le stéarate de magnésium, le talc, le dioxyde de titane et le gel de silice pour produire des granules revêtus;
f) on mélange lesdits granules revêtus avec au moins un agent de viscosité choisi parmi la méthylcellulose, l'hydroxypropylméthylcellulose, la povidone et l'hydroxypropylcellulose et avec du phosphate de calcium ; et
g) on met ledit mélange produit dans l'étape (f) sous la forme d'unités de prise du médicament.

10. Procédé selon la revendication 6 pour la préparation d'une forme pharmaceutique d'un médicament, comprenant :
de 2,0 à 80 % en poids de granules de médicaments revêtus à libération prolongée;
jusqu'à 30 % en poids de phosphate de calcium;
de 2 à 30 % en poids d'un premier agent de viscosité et de 0 à 30 % en poids d'un second agent de viscosité,
dans lequel lesdits premier et second agents de viscosité sont choisis indépendamment parmi la méthylcellulose, l'hydroxypropylméthylcellulose, la povidone et l'hydroxypropylcellulose.

11. Procédé selon la revendication 6 pour la préparation d'une forme pharmaceutique d'un médicament, comprenant:
de 63 à 65 % en poids de granules de divalproex de sodium revêtus à libération prolongée;
de 3,5 à 4,5 % en poids de méthylcellulose;
de 14,2 à 14,6 % en poids d'hydroxypropylméthylcellulose;
de 14,2 à 14,6 % en poids de phosphate de calcium;
de 0,7 à 0,8 % en poids d'acide stéarique ; et
de 1,5 à 2,2 % en poids de talc.

12. Procédé selon la revendication 6 pour la préparation d'une forme pharmaceutique d'un médicament, comprenant:
de 2,6 à 26,4 % en poids de granules de monochlorhydrate de térazosine dihydraté revêtus à libération prolongée;
environ 2,5 % en poids de méthylcellulose;
environ 10,5 % en poids d'hydroxypropylméthylcellulose;
environ 34,0 % en poids de phosphate de calcium;
environ 1,1 % en poids d'acide stéarique ; et
environ 1,9 % en poids de talc.

13. Procédé pour la préparation d'une forme pharmaceutique d'un médicament à libération prolongée, comprenant les étapes suivantes:
a) on enduit des sphères de sucre d'une solution du médicament pour produire des granules de médicament;
b) on mélange lesdits granules avec une composition de revêtement, ladite composition de revêtement comprenant une éthylcellulose ou un ester méthacrylique de méthyle, un plastifiant et un agent antiadhésif, pour produire des granules revêtus;
c) on sèche lesdits granules revêtus ;
d) on tamise et on classe lesdits granules revêtus pour obtenir des granules revêtus de taille désirée;
e) on mélange lesdits granules revêtus de taille désirée avec au moins un agent de viscosité ; et
f) on met ledit mélange produit dans l'étape (e) sous la forme d'unités de prise du médicament,
dans lequel ledit agent de viscosité est choisi parmi la méthylcellulose, l'hydroxypropylméthylcellulose, la povidone et l'hydroxypropylcellulose et la composition de l'étape (e) comprend en outre du phosphate de calcium.

14. Procédé selon la revendication 13, dans lequel ladite composition de revêtement comprend de 2 à 20 % en poids (g/ml x 100) d'une éthylcellulose ou d'un ester méthacrylique de méthyle, de 0,1 à 5,0 % en poids (g/ml x 100) d'un plastifiant et de 0,5 à 20 % en poids (g/ml x 100) d'un agent antiadhésif.

15. Procédé selon la revendication 14, dans lequel ledit plastifiant de l'étape (b) est choisi parmi l'huile de ricin, le propylèneglycol, le polyéthylèneglycol, l'acétylcitrate de triéthyle, l'acétylcitrate de tributyle, le citrate de triéthyle et le citrate de tributyle ; et dans lequel ledit agent antiadhésif de l'étape (b) est choisi parmi le stéarate de magnésium, le talc, le dioxyde de titane et le gel de silice.

16. Procédé selon la revendication 13, dans lequel ledit médicament est choisi parmi le divalproex de sodium, l'acide valproïque ou ses dérivés ou ses sels ; ou la térazosine, ses sels, hydrates ou dérivés.
